# EUROPEAN PATENT SPECIFICATION

(11) **EP 3 908 297 B1**
(45) Date of publication and mention of the grant of the patent: **22.10.2025**
(21) Application number: 20738380.3
(22) Date of filing: 13.01.2020
(51) Int. Cl.: A61K 35/33, C12N 5/00, A61K 35/12, A61K 45/06, A61P 37/02, C12N 5/077

(54) **FIBROBLAST REGENERATIVE CELLS**
REGENERATIVE FIBROBLASTENZELLEN
CELLULES FIBROBLASTES RÉGÉNÉRATRICES

(30) Priority: 11.01.2019 US 201962791207 P
(43) Date of publication of application: 17.11.2021
(73) Proprietor: SpinalCyte LLC, Houston, TX 77289 (US)
(72) Inventor: O'HEERON, Pete, Houston, Texas 77059 (US); ICHIM, Thomas, Houston, Texas 77058 (US)
(74) Representative: J A Kemp LLP
(86) International application number: PCT/US2020/013315
(87) International publication number: WO 2020/146874

(56) References cited:
- WO-A1-2017/135556
- WO-A1-2018/132594
- WO-A1-2018/183653
- WO-A1-2020/097418
- WO-A2-2014/123930
- US-A- 6 008 434
- US-A1- 2010 135 970
- US-A1- 2011 206 644
- US-A1- 2014 349 398
- US-A1- 2015 023 934
- US-A1- 2015 376 570
- US-A1- 2017 101 626
- US-A1- 2017 319 623
- HUANG HSING-I ET AL: "Human foreskin fibroblast-like stromal cells can differentiate into functional hepatocytic cells : Hepatic differentiation of foreskin-derived cells", CELL BIOLOGY INTERNATIONAL., vol. 37, no. 12, 16 September 2013 (2013-09-16), GB, pages 1308 - 1319, XP055920974, ISSN: 1065-6995, DOI: 10.1002/cbin.10175
- HUANG HSING-I ET AL: "Multilineage Differentiation Potential of Fibroblast-like Stromal Cells Derived from Human Skin", TISSUE ENGINEERING PART A, vol. 16, no. 5, 1 May 2010 (2010-05-01), US, pages 1491 - 1501, XP055921120, ISSN: 1937-3341, DOI: 10.1089/ten.tea.2009.0431
- MORSING MIKKEL ET AL: "Evidence of two distinct functionally specialized fibroblast lineages in breast stroma", vol. 18, no. 1, 3 November 2016 (2016-11-03), XP055921336, Retrieved from the Internet <URL:http://link.springer.com/content/pdf/10.1186/s13058-016-0769-2.pdf> DOI: 10.1186/s13058-016-0769-2
- ECKHARD ALT ET AL: "Fibroblasts share mesenchymal phenotypes with stem cells, but lack their differentiation and colony-forming potential", BIOLOGY OF THE CELL, ELSEVIER, PARIS, FR, vol. 103, no. 4, 3 January 2012 (2012-01-03), pages 197 - 208, XP071518583, ISSN: 0248-4900, DOI: 10.1042/BC20100117
- THOMAS E. ICHIM ET AL: "Fibroblasts as a practical alternative to mesenchymal stem cells", JOURNAL OF TRANSLATIONAL MEDICINE, vol. 16, no. 1, 27 July 2018 (2018-07-27), pages 1 - 9, XP055620290, DOI: 10.1186/s12967-018-1536-1
- KAZUTOSHI TAKAHASHI ET AL: "Induction of Pluripotent Stem Cells from Mouse Embryonic and Adult Fibroblast Cultures by Defined Factors", CELL, ELSEVIER, AMSTERDAM NL, vol. 126, no. 4, 25 August 2006 (2006-08-25), pages 663 - 676, XP008157317, ISSN: 0092-8674, DOI: 10.1016/J.CELL.2006.07.024
- PER ANDERSON ET AL: "CD105 (Endoglin)-Negative Murine Mesenchymal Stromal Cells Define a New Multipotent Subpopulation with Distinct Differentiation and Immunomodulatory Capacities", PLOS ONE, vol. 8, no. 10, 4 October 2013 (2013-10-04), pages e76979, XP055407003, DOI: 10.1371/journal.pone.0076979
- DENU RYAN A. ET AL: "Fibroblasts and Mesenchymal Stromal/Stem Cells Are Phenotypically Indistinguishable", vol. 136, no. 2, 1 January 2016 (2016-01-01), CH, pages 85 - 97, XP055828076, ISSN: 0001-5792, Retrieved from the Internet <URL:https://www.karger.com/Article/Pdf/445096> DOI: 10.1159/000445096

## Description

### TECHNICAL FIELD

The present disclosure relates generally to cell biology and medicine. Particularly it concerns regenerative medicine. More particularly, the current disclosure pertains to methods and compositions comprising fibroblasts, fibroblast populations and subpopulations possessing unique regenerative activities.

### BACKGROUND

Regenerative medicine, applied in the form of stem cell therapy, offers the possibility of treating many previously incurable diseases. Numerous types of stem cells exist and there is a need to identify additional stem cells. Generally, stem cells are divided into embryonic and adult types. While embryonic stem cells possess a great ability to proliferate, specific induction of their controlled differentiation has been elusive. The fear of embryonic stem cells causing teratomas has been a major obstacle to their clinical development. Adult stem cells such as bone marrow, cord blood, adipose-derived and amniotic fluid-derived have demonstrated regenerative potential in a variety of diseases and degenerative disorders. However, these cell types are limited by availability, invasiveness of extraction, and in some cases limited proliferative capacity. There is a need for improved cells that overcome these deficiencies and that raise neither issues of oncogenecity nor of karyotypic dysfunction during culture. There is also a need for improved cellular therapies for a variety of medical conditions. The current disclosure overcomes limitations of stem cells by providing a novel regenerative cell, a fibroblast regenerative cell, which possesses superior activity to stem cells. The fibroblast regenerative cell can be utilized for therapeutic purposes.

US 2010/135970 describes methods for reprogramming somatic cells and related therapeutic compositions and methods. WO 2014/123930 describes methods for preventing development and/or treatment of tissue fibrosis through administration of stem cells. WO 2018/183653 describes methods of reprogramming mammalian fibroblasts to hemangioblast-like CD34-postive mesodermal progenitor cells. US 6008434 describes a transgenic mouse with disruption of endogenous growth differentiation factor-11 (GDF-11). US 2017/101626 describes a method of obtaining a cardiac multipotent or pluripotent cell. US 2015/376570 describes methods and compositions for use in generating induced hepatocytes by reprogramming non-hepatocyte cells. US 2014/349398 describes methods for providing endothelial cells and precursors of endothelial cells from a variety of cell sources, such as pluripotent stem cells. US 2011/206644 describes methods for affecting and/or altering the differentiation state of a cell. US 2017/319623 describes a quiescent stem cell having the capacity to differentiate into ectoderm, mesoderm and endoderm, and which does not express cell surface markers including MHC class I, MHC class II, CD44, CD45, CD13, CD34, CD49c, CD73, CD105 and CD90. US 2015/023934 describes methods for reprogramming fibroblast cells in culture, which are able to generate generic epithelial cells therefrom. WO 2017/135556 describes a medium composition for culturing fibroblasts which contain GDF11 or a human-derived adult stem cell culture fluid comprising the same; a method for culturing fibroblasts by using the medium composition; and a method for producing GDF11 by culturing the stem cell. WO 2018/132594 describes compositions of matter, cells, protocols and procedures useful for augmentation of one or more therapeutic activities of fibroblast cellular populations. WO 2020/097418 describes methods and compositions using fibroblasts for stimulating regeneration in a first tissue site in an individual, comprising the step of administering at least one regenerative composition to a second tissue site, wherein the second tissue site comprises the same tissue type as the first tissue site in the individual. Huang Hsing et al 2013 (Cell Biology International., vol. 37, no. 12, 16 September 2013, pages 1308-1319) describes human foreskin fibroblast-like stromal cells can differentiate into functional hepatocytic cells. Huang Hsing et al 2010 (Tissue Engineering Part A, vol. 16, no. 5, 1 May 2010) describes multilineage differentiation potential of fibroblast-like stromal cells derived from human skin. Morsing Mikkel et al 2016 (Breast Cancer Research, vol. 18, no. 1,3 November 2016) describes two distinct functionally specialized fibroblast lineages in breast stroma. Eckhard Alt et al 2012 (Biology of the Cell, Elsevier, Paris, France, vol. 103, no. 4, 3 January 2012) describes that fibroblasts share mesenchymal phenotypes with stem cells, but lack their differentiation and colony-forming potential. Thomas Ichim et al 2018 (Journal of Translational Medicine, vol. 16, no. 1,27 July 2018, pages 1 -9) describes fibroblasts as a practical alternative to mesenchymal stem cells. Kazutoshi Takahashi et al 2006 (Cell Elsevier, Amsterdam, NL, vol. 126, no. 4, 25 August 2006, pages 663-676) describes induction of pluripotent stem cells from mouse embryonic and adult fibroblast cultures by defined factors. Per Anderson et al 2012 (PLOS ONE, vol. 8, no. 10, 4 October 2013, page e76979) describes CD105-negative murine mesenchymal stromal cells define a new multipotent subpopulation with distinct differentiation and immunomodulatory capacities. Denu Ryan et al 2016 (Acta Haematologica, vol. 136, no. 2, 1 January 2016, pages 85-97) describes fibroblasts and mesenchymal stromal cells are phenotypically indistinguishable.

### BRIEF SUMMARY

The invention provides an *in vitro* method of producing a fibroblast regenerative cell or a population thereof, the method comprises selecting and expanding a fibroblast cell that expresses CD105 and further comprises a step of selecting the fibroblast regenerative cell for GDF-3, wherein the fibroblast cell has increased regenerative activity as compared to a control fibroblast cell that does not express CD105.

The invention further provides a fibroblast regenerative cell obtainable by the *in vitro* method for use in therapy, wherein a therapeutically effective amount of the fibroblast regenerative cell, or population thereof is provided to a subject in need thereof.

The invention also provides a composition comprising an isolated fibroblast cell, or a population thereof wherein the fibroblast cell has an increased regenerative activity compared to a control fibroblast cell wherein the fibroblast cell expresses CD105 marker and GDF-3 and the control fibroblast cell does not express CD105.

The invention also provides a kit comprising the composition.

The invention also provides a pharmaceutical formulation comprising the composition.

The invention is defined in the appended claims. Any aspects, embodiments and examples of the present disclosure which do not fall under the scope of the appended claims do not form part of the invention and are merely provided for illustrative purposes. The references to methods of treatment in paragraphs [4f], [8], [10], [12]-[18], [33], [39], [67], [92], [99], [101]-[120], [124] and [125] of this description are to be interpreted as references to the compounds, pharmaceutical compositions and medicaments of the present invention for use in a method of treatment of the human (or animal) body by therapy (or diagnosis).

### SUMMARY OF TECHNICAL TEACHINGS

Aspects of the disclosure encompass compositions, methods, and systems comprising fibroblasts possessing regenerative activity that are useful in cellular therapy and in the treatment of various diseases, disorders or conditions. In some aspects, the fibroblasts have enhanced proliferative potential based on enrichment at least for one or more markers. In specific aspects, the fibroblasts express CD105 and/or CD117 markers. In some aspects, fibroblast regenerative cells possessing CD105 and/or CD117 markers are further enriched for the property of rhodamine 123 efflux. In some aspects, the fibroblast regenerative cells do not express at least one or more of MHC class I, MHC class II, CD45, CD13, CD49c, CD66b, CD73, CD105 or CD90 cell surface proteins.

Aspects include compositions comprising fibroblast regenerative cells, a population, progeny or subset of fibroblast regenerative cells, a conditioned medium of fibroblast regenerative cells, compositions comprising fibroblast regenerative cells, pharmaceutical formulations comprising fibroblast regenerative cells, kits comprising fibroblast regenerative cells, a cell bank comprising populations of fibroblast regenerative cells, and so forth.

In some aspects, the isolated fibroblast regenerative cells express or are selected because they express CD105 marker and/or CD117 marker. In some aspects, the cells also express Oct-4, CD-34, KLF-4, Nanog, Sox-2, Rex-1, GDF-3, IL-10, Stella and/or any combination thereof. In some aspects fibroblast regenerative cells also comprise a rhodamine 123 efflux activity. In some aspects, the cells comprise enhanced expression of GDF-11 as compared to a control cell.

In some aspects of the current disclosure, the fibroblast regenerative cells are derived from a tissue having regenerative properties such as, but not limited to, placental tissue, umbilical cord tissue, endometrial cells, Wharton's jelly, bone marrow, adipose tissue, or a mixture thereof. In some aspects, the regenerative fibroblast cells are capable of proliferating and differentiating into at least two of ectoderm, mesoderm, or endoderm. The fibroblast regenerative cells may be autologous or allogeneic with respect to a subject undergoing treatment. In some aspects, the cells are plastic adherent. In further aspects, the cells may be cryopreserved.

Further aspects of the disclosure relate to methods or compositions comprising fibroblasts regenerative cells and exogenous mitochondria. In some aspects, the exogenous mitochondria are isolated and substantially purified from other cellular components. In some aspects, the mitochondria are administered to the fibroblast regenerative cells by lipid fusion, polyethylene glycol-mediated fusion, or by electroporation. In some aspects, the mitochondria are encapsulated or treated with one or more agents to facilitate the incorporation of the mitochondria into the fibroblast cell. The mitochondria can be autologous, syngeneic, allogeneic, or xenogeneic with respect to a subject undergoing treatment. In some aspects, the mitochondria are derived from stem cells, or from cells less differentiated as compared to the fibroblast regenerative cell. In some methods, the mitochondria are administered separately to the subject.

The current disclosure also relates to an isolated fibroblast regenerative cell or population thereof capable of proliferating and differentiating into ectoderm, mesoderm, or endoderm, wherein the isolated fibroblast regenerative cell expresses at least one of Oct-4, Nanog, IL-10, Sox-2, KLF4, c-Myc, Rex-1, GDF-3, LIF receptor, CD105, CD117, CD344 or Stella markers, and does not express at least one of MHC class I, MHC class II, CD45, CD13, CD49c, CD66b, CD73, CD105, or CD90 cell surface proteins.

In certain aspects, the cells, compositions or formulations of the current disclosure are administered with one or more other compounds that are not fibroblast regenerative cells, a population thereof, progeny thereof and/or a conditioned medium thereof. The one or more other compounds may or may not be in the cells, compositions or formulations encompassed herein. In specific cases, the compounds can be vitamin A, vitamin C, vitamin D, vitamin E, vitamin K, folic acid, choline, vitamin B1, vitamin B2, vitamin B5, vitamin B6, vitamin B12, biotin, nicotinamide, betacarotene, coenzyme Q, selenium, superoxide dismutase, glutathione peroxide, uridine, creatine succinate, pyruvate, dihydroxyacetone), acetyl-L-carnitine, alpha-lipoic acid, cardiolipin, omega fatty acid, lithium carbonate, lithium citrate, calcium, or any combination thereof. In some aspects, the compounds comprise one or more anti-inflammatory agents. In some aspects, the anti-inflammatory agents are one or more of Alclofenac; Alclometasone Dipropionate; Algestone Acetonide; Alpha Amylase; Alpha-lipoic acid; Alpha tocopherol; Amcinafal; Amcinafide; Amfenac Sodium; Amiprilose Hydrochloride; Anakinra; Anirolac; Anitrazafen; Apazone; Ascorbic Acid; Balsalazide Disodium; Bendazac; Benoxaprofen; Benzydamine Hydrochloride; Bromelains; Broperamole; Budesonide; Carprofen; Chlorogenic acid; Cicloprofen; Cintazone; Cliprofen; Clobetasol Propionate; Clobetasone Butyrate; Clopirac; Cloticasone Propionate; Cormethasone Acetate; Cortodoxone; Deflazacort; Desonide; Desoximetasone; Dexamethasone Dipropionate; Diclofenac Potassium; Diclofenac Sodium; Diflorasone Diacetate; Diflumidone Sodium; Diflunisal; Difluprednate; Diftalone; Dimethyl Sulfoxide; Drocinonide; Ellagic acid; Endrysone; Enlimomab; Enolicam Sodium; Epirizole; Etodolac; Etofenamate; Felbinac; Fenamole; Fenbufen; Fenclofenac; Fenclorac; Fendosal; Fenpipalone; Fentiazac; Flazalone; Fluazacort; Flufenamic Acid; Flumizole; Flunisolide Acetate; Flunixin; Flunixin Meglumine; Fluocortin Butyl; Fluorometholone Acetate; Fluquazone; Flurbiprofen; Fluretofen; Fluticasone Propionate; Furaprofen; Furobufen; Glutathione; Halcinonide; Halobetasol Propionate; Halopredone Acetate; Hesperedin; Ibufenac; Ibuprofen; Ibuprofen Aluminum; Ibuprofen Piconol; Ilonidap; Indomethacin; Indomethacin Sodium; Indoprofen; Indoxole; Intrazole; Isoflupredone Acetate; Isoxepac; Isoxicam; Ketoprofen; Lofemizole Hydrochloride; Lomoxicam; Loteprednol Etabonate; Lycopene; Meclofenamate Sodium; Meclofenamic Acid; Meclorisone Dibutyrate; Mefenamic Acid; Mesalamine; Meseclazone; Methylprednisolone Suleptanate; Morniflumate; Nabumetone; Naproxen; Naproxen Sodium; Naproxol; Nimazone; Oleuropein; Olsalazine Sodium; Orgotein; Orpanoxin; Oxaprozin; Oxyphenbutazone; Paranyline Hydrochloride; Pentosan Polysulfate Sodium; Phenbutazone Sodium Glycerate; Pirfenidone; Piroxicam; Piroxicam Cinnamate; Piroxicam Olamine; Pirprofen; Pycnogenol; Polyphenols; Prednazate; Prifelone; Prodolic Acid; Proquazone; Proxazole; Proxazole Citrate; Quercetin; Reseveratrol; Rimexolone; Romazarit; Rosmarinic acid; Rutin; Salcolex; Salnacedin; Salsalate; Sanguinarium Chloride; Seclazone; Sermetacin; Sudoxicam; Sulindac; Suprofen; Talmetacin; Talniflumate; Talosalate; Tebufelone; Tenidap; Tenidap Sodium; Tenoxicam; Tesicam; Tesimide; Tetrahydrocurcumin; Tetrydamine; Tiopinac; Tixocortol Pivalate; Tolmetin; Tolmetin Sodium; Triclonide; Triflumidate; Zidometacin; Zomepirac Sodium. In some aspects, the compounds are bioactive compounds including but not limited to growth factors, cytokines, antibodies, antibody fragments, and/or organic molecules, for example of a mass of less than 5000 daltons. The compounds referred to above or elsewhere herein may or may not be administered concurrently with a composition of the current disclosure. Alternatively, the compounds may be administered before and/or after the composition is administered to a subject.

In some aspects, a composition comprising fibroblasts further comprises one or more types of stem cells, including embryonic stem cells, tissue-specific stem cells, mesenchymal stem cells, or induced pluripotent stem cells, for example. The cells may be provided in the amounts of at least 1x10², 1x10⁶, 1x10⁹, 1x10¹⁰, 1x10¹², 1x10¹⁴ stem cells or any amount in between. The stem cells can be mesenchymal cells, embryonic stem cells or differentiated cells. For example, the stem cells can be myocytes, adipocytes, ectodermal cells, muscle cells, osteoblasts, chondrocytes, endothelial cells, fibroblasts, pancreatic cells, hepatocytes, bile duct cells, bone marrow cells, neural cells, or genitourinary cells, as examples.

Aspects also include methods of providing cell therapy, methods of treating a subject who would benefit from cell therapy, methods of treating a disease, a disorder or a condition, methods of treating cancer, methods of treating degenerative diseases, methods of transplanting fibroblast regenerative cells, methods for using fibroblast regenerative cells, methods of enhancing the regenerative activity of fibroblast cells, method of generating a population of fibroblast regenerative cells, methods of producing fibroblast regenerative cells, methods for selecting for a fibroblast regenerative cell, methods for identifying a fibroblast regenerative cell, methods for isolating a population of regenerative fibroblast cells, methods of culturing, methods of enriching fibroblast regenerative cells, methods of expanding fibroblast regenerative cells, or a combination thereof. The steps and aspects discussed in this disclosure are contemplated as part of any of these methods. Moreover, compositions for use in any of these methods are also contemplated.

Particular aspects include methods of treating at least one condition in a subject by administering to the subject a composition comprising isolated fibroblast regenerative cells, a population thereof, a progeny thereof, and/or a conditioned medium thereof, wherein the fibroblast regenerative cells have an increased regenerative activity compared to a control fibroblast cell.

Methods may comprise or consist of or consist essentially of one or more of the following steps: administering to a subject an effective amount of fibroblast regenerative cells, and administering to the subject one or more additional compounds that enhance or increase or ameliorate the regenerative activity of the fibroblasts of the current disclosure.

In some aspects, the subject suffers from or is at risk of developing or is suspected of having a condition, disease or disorder. In some aspects, the subject may have undergone one or more previous treatments for the condition, disease or disorder. In further aspects, the subject may be resistant to certain other therapies. The condition, disease or disorder may be an immune disorder, a muscular disorder, a hematopoietic disorder, a liver disorder, an angiogenesis disorder, a pancreatic disorder, a cardiac disease, a pulmonary disease, neurological disease, neurological disorder, neurodegenerative disease, muscular disorder, muscular disease, immune disease, inflammatory-mediated disease, inflammatory-mediated disorder, inflammation, ischemia, stroke, ischemic heart disease, liver failure, kidney failure, peripheral artery disease, pulmonary fibrosis, liver fibrosis, pancreatic fibrosis, diabetic limb, fibrosis, scar tissue formation, pathological apoptosis, diabetes, cirrhosis, hepatitis, osteoporosis, a bone fracture, a neurological injury, the need for a prosthesis in a joint, the need for dermal stem cells, the need to increase the proliferation of islet cells, the need to increase proliferation of hepatocytes, the need to increase insulin production, the need for osteocytes, the need to increase osteocyte formation, the need to increase osteocyte function, the need for a neoplastic disease, or the need of cell therapy. In some aspects, the cardiac disease or pulmonary disease is atherosclerosis, myocardial infarction (Heart Attack), cardiac infection, heart failure, ischemic heart failure, high blood pressure (Hypertension), or pulmonary hypertension, idiopathic pulmonary fibrosis, stroke, congenital heart disease (CHD), congestive heart failure, angina, myocarditis, coronary artery disease, cardiomyopathy, dilated cardiomyopathy, hypertrophic cardiomyopathy, endocarditis, diastolic dysfunction, cerebrovascular disease, valve disease, mitral valve prolapse, venous thromboembolism or arrhythmia. In some aspects, the neurological or muscular disease is multiple sclerosis (MS), spinal cord injury, muscular dystrophy (Becker's or Duchenne's), amyotrophic lateral sclerosis (ALS; Lou Gehrig's disease or classical motor neuron disease), autism, progressive bulbar palsy (progressive bulbar atrophy), pseudobulbar palsy, primary lateral sclerosis (PLS), progressive muscular atrophy, spinal muscular atrophy (SMA, including SMA type I--Werdnig-Hoffmann disease, SMA type II, or SMA type III-Kugelberg-Welander disease), Fazio-Londe disease, Kennedy disease (progressive spinobulbar muscular atrophy), congenital SMA with arthrogryposis, and post-polio syndrome (PPS). In some aspects, the immune disorder or the inflammatory-mediated disorder or the immune disease or the inflammatory-mediated disease is thyroiditis, insulitis, multiple sclerosis, iridocyclitis, uveitis, orchitis, Addison's disease, myasthenia gravis, rheumatoid arthritis, lupus erythematosus, immune hyperreactivity, insulin dependent diabetes mellitus, anemia, aplastic anemia, hemolytic anemia, hepatitis, autoimmune hepatitis, skleritis, idiopathic thrombocytopenic purpura, auto immune diseases, diseases of the gastrointestinal tract, Crohn's disease, ulcerative colitis, inflammatory bowel diseases, juvenile arthritis, scleroderma and systemic sclerosis, Sjogren's syndrome, undifferentiated connective tissue syndrome, antiphospholipid syndrome, vasculitis, *polyarteritis nodosa,* allergic granulomatosis, angiitis, Wegner's granulomatosis, Kawasaki disease, hypersensitivity vasculitis, Henoch-Schoenlein purpura, Behcet's Syndrome, Takayasu arteritis, Giant cell arteritis, Thrombangiitis obliterans, polymyalgia rheumatica, essentiell cryoglobulinemia, *Psoriasis vulgaris* and psoriatic arthritis, diffuse fasciitis with eosinophilia, diffuse fasciitis without eosinophilia, polymyositis and other idiopathic inflammatory myopathies, relapsing panniculitis, relapsing polychondritis, lymphomatoid granulomatosis, erythema nodosum, ankylosing spondylitis, Reiter's syndrome, inflammatory dermatitis, unwanted immune reactions and inflammation associated with arthritis, rheumatoid arthritis, inflammation associated with hypersensitivity and allergic reactions, systemic lupus erythematosus, collagen diseases, inflammation associated with atherosclerosis, arteriosclerosis, atherosclerotic heart disease, reperfusion injury, vascular inflammatory disorders, respiratory distress syndrome, cardiopulmonary diseases, inflammation associated with peptic ulcer, hepatic fibrosis, liver cirrhosis, hepatic diseases, thyroiditis or other glandular diseases, glomerulonephritis, renal diseases, urologic diseases, otitis, oto-rhino-laryngological diseases, dermatitis, dermal diseases, periodontal diseases, dental diseases, orchitis or epididimo-orchitis, infertility, orchidal trauma, immune related testicular diseases, placental dysfunction, placental insufficiency, habitual abortion, eclampsia, pre-eclampsia, immune-related gynaecological diseases, inflammatory-related gynaecological diseases, posterior uveitis, intermediate uveitis, anterior uveitis, conjunctivitis, chorioretinitis, uveoretinitis, optic neuritis, intraocular inflammation, e.g. retinitis or cystoid macular oedema, sympathetic ophthalmia, scleritis, retinitis pigmentosa, immune and inflammatory components of degenerative fondus disease, inflammatory components of ocular trauma, ocular inflammation caused by infection, proliferative vitreo-retinopathies, acute ischaemic optic neuropathy, excessive scarring, immune reaction to ocular implants, inflammation reaction against ocular implants, immune-related ophthalmic diseases, inflammatory-related ophthalmic diseases, inflammation associated with autoimmune diseases or conditions or disorders of the central nervous system (CNS) or in any other organ, Parkinson's disease, complication and/or side effects from treatment of Parkinson's disease, AIDS-related dementia complex HIV-related encephalopathy, Devic's disease, Sydenham chorea, Alzheimer's disease and other degenerative diseases, conditions or disorders of the CNS, inflammatory components of strokes, post-polio syndrome, immune and inflammatory components of psychiatric disorders, myelitis, encephalitis, subacute sclerosing pan-encephalitis, encephalomyelitis, acute neuropathy, subacute neuropathy, chronic neuropathy, Guillaim-Barre syndrome, Sydenham chora, pseudo-tumour cerebri, Down's Syndrome, Huntington's disease, amyotrophic lateral sclerosis, inflammatory components of CNS compression, inflammatory components of CNS compression, inflammatory components of CNS compression, CNS trauma infections of the CNS, inflammatory components of muscular atrophies, inflammatory components of muscular dystrophies, conditions or disorders of the central and peripheral nervous systems, post-traumatic inflammation, septic shock, infectious diseases, inflammatory complications of surgery, inflammatory complications of organ transplant, side effects of surgery, side effects of organ transplants, inflammatory complications of gene therapy, immune complications of gene therapy, inflammatory-related side effects of gene therapy, immune-related side effects of gene therapy, inflammation associated with AIDS, humoral immune response, cellular immune response, monocyte proliferative disease, leukocyte proliferative diseases, leukemia, high amounts of monocytes or lymphocytes, or graft rejection. In some aspects, the graft rejection is after transplantation of natural cells, artificial cells, a natural tissue, an artificial tissue, bone marrow, an organ, a pacemaker, a cornea or a lens. In some aspects, the organ is a liver, a kidney, a heart, or a lung. In some aspects, the neurodegenerative disease is Parkinson's disease, Alzheimer's disease, or Huntington disease. In some aspects, the cardiac or pulmonary disease is artherosclerosis, myocardial infarction, cardiac infection, heart failure, ischemic heart failure, hypertension, pulmonary hypertension, idiopathic pulmonary fibrosis, stroke, congenital heart disease (CHD), congestive heart failure, angina, myocarditis, coronary artery disease, cardiomyopathy, dilated cardiomyopathy, hypertrophic cardiomyopathy, endocarditis, diastolic dysfunction, cerebrovascular disease, valve disease, mitral valve prolapse, venous thromboembolism or arrhythmia.

In some aspects, the subject is in need of increased hematopoiesis, increased liver activity, increased angiogenesis, controlled inflammation, controlled autoimmunity, and/or has ischemia. The ischemia may be in a cardiac tissue, a pulmonary tissue, a kidney tissue, a brain tissue, muscle tissue, in a limb, or the ischemia may be associated with stroke, ischemic heart disease, liver failure, kidney failure, and peripheral artery disease. In some aspects, the subject has or is at risk of developing a stroke, pulmonary fibrosis, a diabetic limb, an ischemic heart disease, liver failure, kidney failure, peripheral artery disease, diabetes, liver failure, cirrhosis, liver or pancreas fibrosis, or hepatitis, osteoporosis, bone fracture, cardiac disease, pulmonary disease or scar tissue formation. In some aspects, the scar tissue formation or fibrosis is in a pancreatic tissue, a liver tissue, a cardiac tissue, a pulmonary tissue, a limb, liver, pancreas or kidney.

In some aspects, the subject is in need of inhibition, reduction, controlled or reversal of or decreased pathological apoptosis. In some aspects, the subject is in need of improved pancreas function, liver function, osteocyte function, insulin production, pulmonary function, cardiac function or is in need of a prosthesis in a joint, increased number of islet cells, hepatocytes, increased insulin production; and/or an increased number of osteocytes. In some aspects, the subject is in need of dermal stem cells and/or activation of endogenous dermal stem cells. In further aspects, the subject is an animal.

In some aspects of the methods of the current disclosure, the compositions comprising fibroblast regenerative cells are introduced to the subject parenterally, transdermally, transmucosally, by implantation or by transplantation. For example the compositions are administered intravenously, intramuscularly, intrathecally, intrarterially, intradermally, subcutaneously, intra-pleurally, intra-cranially, intra-ocularly or mucosally.

Further aspects of the disclosure relate to an *in vitro* method of producing a fibroblast regenerative cell or a population thereof, the method comprising selecting and/or expanding a fibroblast cell that expresses CD117 and/or CD105, wherein the fibroblast cell has increased regenerative activity as compared to a control fibroblast cell, including in at least some cases a fibroblast cell that does not express CD117 and/or CD105. In certain aspects, the method further comprises a step of selecting the fibroblast regenerative cell for CD34 expression. In other aspects, the method further comprises a step of selecting the fibroblast regenerative cell for rhodamine 123 efflux activity. In some aspects, the method further comprises a step of selecting the fibroblast regenerative cell for at least one additional marker selected from the group consisting of Oct-4, CD-34, KLF-4, Nanog, Sox-2, Rex-1, GDF-3, Stella, IL-10, and a combination thereof. In yet some additional aspects the method further comprises a step of selecting the fibroblast regenerative cell for enhanced expression of GDF-11 as compared to a control fibroblast cell. In some aspects of the method, the fibroblast regenerative cell does not express at least one or more of MHC class I, MHC class II, CD45, CD13, CD49c, CD66b, CD73, CD105 or CD90 cell surface proteins. In some aspects, the method further comprises the step of preparing a therapeutically effective amount of the fibroblast regenerative cell and providing the therapeutically effective amount of the fibroblast regenerative cell, population thereof, progeny thereof, or conditioned medium thereof to a subject in need thereof. In some aspects, the population of fibroblast regenerative cells comprises at least 0.01% to 5% freshly extracted fibroblasts.

Further aspects of the disclosure relate to an *in vitro* method of forming or regenerating one or more neural cells, comprising the steps of: obtaining a fibroblast regenerative cell or a population thereof by selecting a fibroblast cell that expresses CD117 and/or CD105, wherein the fibroblast cell has increased regenerative activity as compared to a control fibroblast cell; and culturing the fibroblast regenerative cell with a combination of at least two of bFGF, FGF-8, SHH, or BDNF.

In other aspects, the disclosure relates to an *in vitro* method of forming a hepatocyte cell, comprising the steps of: obtaining a fibroblast regenerative cell or a population thereof by selecting and expanding a fibroblast cell that expresses CD117 and/or CD105, wherein the fibroblast cell has increased regenerative activity as compared to a control fibroblast cell; and culturing said fibroblast regenerative cell with hepatocyte growth factor (HGF) and/or FGF-4.

In other aspects, the disclosure relate to an *in vitro* method of forming an endothelial cell, comprising the steps of: obtaining a fibroblast regenerative cell or a population thereof by selecting a fibroblast cell that expresses CD117 and/or CD105, wherein the fibroblast cell has increased regenerative activity as compared to a control fibroblast cell; and culturing said fibroblast regenerative cell with vascular endothelial growth factor (VEGF).

The disclosure also relates to an *in vitro* method of forming a hematopoietic cell, comprising the steps of: obtaining a fibroblast regenerative cell or a population thereof by selecting a fibroblast cell that expresses CD117 and/or CD105, wherein the fibroblast cell has increased regenerative activity as compared to a control fibroblast cell; and culturing said fibroblast regenerative cell with a combination of at least two of bone morphogenic protein-4 (BMP4), VEGF, bFGF, stem cell factor (SCF), Flt3L, hyper IL6, thrombopoietin (TPO) or erythropoietin (EPO).

In certain aspects of the above *in vitro* methods, the method further comprises a step of selecting the fibroblast regenerative cell for CD34 expression and/or the method further comprises a step of selecting the fibroblast regenerative cell for rhodamine 123 efflux activity. In some aspects, the above methods further comprise a step of selecting the fibroblast regenerative cell for at least one additional marker selected from the group consisting of Oct-4, CD-34, KLF-4, Nanog, Sox-2, Rex-1, GDF-3, Stella, IL-10, and a combination thereof. In some aspects, the methods further comprise a step of selecting the fibroblast regenerative cell for enhanced expression of GDF-11 as compared to a control fibroblast cell. In further aspects of the above methods, the fibroblast regenerative cell does not express at least one or more of MHC class I, MHC class II, CD45, CD13, CD49c, CD66b, CD73, CD105 or CD90 cell surface proteins.

Further aspects of the current disclosure relate to a composition and/or pharmaceutical formulations comprising an isolated fibroblast cell, a population thereof, a progeny thereof, or a conditioned medium thereof, wherein the fibroblast cell has an increased regenerative activity compared to a control fibroblast cell. In some aspects, the fibroblast cell expresses CD105 marker and/or CD117 marker. In some aspects, the fibroblast cell further comprises a rhodamine 123 efflux activity and/or further expresses at least one additional marker selected from the group consisting of Oct-4, CD-34, KLF-4, Nanog, Sox-2, Rex-1, GDF-3, Stella, IL-10, and a combination thereof. The fibroblast cell may have enhanced expression of GDF-11 as compared to a control cell. In some aspects, the population comprises at least 1x10², 1x10⁶, 1x10⁹, 1x10¹⁰, 1x10¹², 1x10¹⁴ cells or any amount in between. In some aspects, the composition is in a NaCl solution, such as a NaCl solution that is 0.8%-1% NaCl.

In some aspects of the current disclosure, the regenerative activity of fibroblast regenerative cells is the ability to stimulate angiogenesis. In some aspects, angiogenesis further comprises stimulation of human umbilical vein endothelial cell (HUVEC) proliferation. In some aspects, angiogenesis comprises the production of collateral blood vessels. The collateral blood vessels can be an ischemic cardiac tissue or in an ischemic limb tissue or surrounding an occluded blood vessel.

The current disclosure further provides kits comprising any of the above fibroblast regenerative cells, populations, compositions, progeny, conditioned media or formulations thereof.

In further aspects, the disclosure provides a master cell bank comprising a plurality of packaged population of regenerative fibroblast cells, capable of proliferating and differentiating into ectoderm, mesoderm, or endoderm, wherein the isolated fibroblast regenerative cell expresses at least one of Oct-4, Nanog, Sox-2, KLF4, c-Myc, Rex-1, GDF-3, LIF receptor, CD105, CD117, CD344, IL-10, and Stella, and does not express at least one of MHC class I, MHC class II, CD45, CD13, CD49c, CD66b, CD73, CD105, or CD90 cell surface proteins. In some aspects, the packaged population includes at least 1x10² or more of the cells of previous aspect.

The current disclosure also relates to aspects for a method for isolating a population of regenerative fibroblast cells, the method comprising providing a tissue with regenerative activity; and enriching for a population of cells that are about 6-12 micrometers in size, wherein the fibroblast regenerative cells express at least one of Oct-4, Nanog, Sox-2, KLF4, c-Myc, Rex-1, GDF-3, LIF receptor, CD105, CD117, CD344, IL-10, and Stella, and does not express at least one of MHC class I, MHC class II, CD45, CD13, CD49c, CD66b, CD73, CD105, or CD90 cell surface proteins. In some aspects, the method optionally includes the step of depleting cells from the population expressing stem cell surface markers or MHC proteins, thereby isolating a population of stem cells. In some aspects, the cells to be depleted express MHC class I, CD66b, glycophorin a, or glycophorin b. In some aspects, the method optionally includes transfecting the cells with a polynucleotide vector containing a stem cell-specific promoter operably linked to a reporter or selection gene. In some aspects, the cell-specific promoter is an Oct-4, Nanog, Sox-9, GDF3, Rex-1, or Sox-2 promoter. In some aspects, the method further includes the step of enriching the population for the regenerative fibroblast cells using expression of a reporter or selection gene. In some aspects, the method further includes the step of enriching the population of the regenerative fibroblast cells by flow cytometry. In some aspects, the method further includes the steps of contacting the cells with a detectable compound that enters the cells, the compound being selectively detectable in proliferating and non-proliferating cells; and enriching the population of cells for the proliferating cells. In some aspects, the detectable compound is carboxyfluorescein diacetate, succinimidyl ester, or Aldefluor. In some aspects, method further includes culturing the cells under conditions that form tissue aggregate bodies. In some aspects, the method further includes culturing the population of fibroblast regenerative cells under conditions that support proliferation of the cells.

In some aspects, the method further includes separating cell types such as granulocytes, T-cells, B-cells, NK-cell, red blood cells, or any combination thereof, from the fibroblast regenerative cells. In some aspects, separating the cell types is done by cell depletion.

Further aspects of the current disclosure relate to a method of identifying a fibroblast regenerative cell, the method comprises the steps of introducing into a cell a vector comprising a fibroblast cell-specific promoter coupled to at least one selectable marker gene; expressing the selectable marker gene from the cell specific promoter in the cell; and detecting expression of the marker gene in the cell, thereby identifying the fibroblast regenerative cell, wherein said fibroblast regenerative cell does not express at least one or more of MHC class I, MHC class II, CD44, CD45, CD13, CD34, CD49c, CD66b, CD73, CD105, and CD90 cell surface proteins; and said fibroblast regenerative cell expresses at least one or more of Oct-4, Nanog, Sox-2, Rex-1, GDF-3, Stella, FoxD3, IL-10, or Polycomb embryonic transcription factors, and wherein said fibroblast regenerative cell is capable of differentiating into mesoderm, ectoderm, and/or endoderm. In some aspects, the fibroblast cell does not express CD13, CD44, CD90, or a combination thereof.

In some aspects, the fibroblast cell-specific promoter is an Oct-4 promoter, a Nanog promoter, a Sox-2 promoter, a Rex-1 promoter, a GDF-3 promoter, a Stella promoter, a FoxD3 promoter, a Polycomb Repressor Complex 2 promoter, an IL-10 promoter, or aCTCF promoter. In some aspects, the fibroblast cell-specific promoter is flanked by loxP sites.

In some aspects, the method further comprises the step of isolating the fibroblast regenerative cell. In some aspects the fibroblast regenerative cell is derived from the bodily fluid and/or from the tissue of a mammal. In some aspects, the bodily fluid is synovial fluid or blood. In some aspects, the mammal is a human.

In some aspects, the vector is a retroviral vector. In some aspects, the selectable marker gene encodes a fluorescent protein, such as but limited to Green Fluorescent Protein (GFP). In some aspects, the vector comprises two selectable marker genes, the two selectable marker genes comprise a fluorescent protein, a protein sensitive to drug selection, a cell surface protein or any combination thereof.

Further aspects of the disclosure relate to a method of generating a regenerative fibroblast cell comprising the steps of introducing into a population of fibroblasts a vector comprising a promoter, such as a regenerative cell-specific promoter, coupled to at least one selectable marker gene, wherein said regenerative cell does not express MHC class I, MHC class II, CD44, CD45, CD13, CD34, CD49c, CD73, CD105 and CD90 cell surface proteins; expressing the selectable marker gene from the regenerative-cell specific promoter in said fibroblast population; and detecting expression of the marker gene in the regenerative fibroblast cell.

In some aspects, the methods further comprise a step of transfecting the regenerative fibroblast cells with OCT-4 transcription factor, thereby enhancing the regenerative activity of the fibroblast cells.

In some aspects, the methods further comprise a step of fusing the regenerative fibroblast cells with cells having a pluripotent ability thereby generating fibroblasts with enhanced regenerative activity.

In some aspects, the methods further comprise the steps of selecting fibroblast cells expressing CD105 and/or CD117; and transfecting the fibroblast cells expressing CD105 and/or CD117 with permeant NANOG gene. In some aspects, the fibroblast regenerative cell further comprises a rhodamine 123 efflux activity. In further aspects, the fibroblast regenerative cell has enhanced expression of GDF-11 as compared to a control

Use of the one or more compositions may be employed based on methods described herein. Use of one or more compositions may be employed in the preparation of medicaments for treatments according to the methods described herein. Other aspects are discussed throughout this application. Any aspect discussed with respect to one aspect of the disclosure applies to other aspects of the disclosure as well and vice versa.

The novel features which are believed to be characteristic of the designs disclosed herein, both as to the organization and method of operation, together with further objects and advantages will be better understood from the following description when considered in connection with the accompanying figures.

### BRIEF DESCRIPTION OF THE DRAWINGS

For a more complete understanding of the present disclosure, reference is now made to the following descriptions taken in conjunction with the accompanying drawings.
FIG. 1 shows enhanced expression of OCT-4 in CD105 purified fibroblasts. Control is the left bar, CD105-negative is the middle bar, and CD105-positive is the right bar.
FIG. 2 demonstrates enhanced expression of NANOG in CD105 purified fibroblasts. Control is the left bar, CD105-negative is the middle bar, and CD105-positive is the right bar.
FIG. 3 shows enhanced expression of KLF-4 in CD105 purified fibroblasts. Control is the left bar, CD105-negative is the middle bar, and CD105-positive is the right bar.
FIG. 4 demonstrates enhanced expression of Immune modulatory cytokine IL-10 by CD105 selected fibroblasts. Control is the left bar, CD105-negative is the middle bar, and CD105-positive is the right bar.

### DETAILED DESCRIPTION

### Definitions

In keeping with long-standing patent law convention, the words "a" and "an" when used in the present specification in concert with the word comprising, including the claims, denote "one or more." Some aspects of the disclosure may consist of or consist essentially of one or more elements, method steps, and/or methods of the disclosure. It is contemplated that any method or composition described herein can be implemented with respect to any other method or composition described herein and that different aspects may be combined.

As used herein, the term "cell surface protein" refers to a protein that is expressed on the surface of a cell.

As used herein, the term "expansion" refers to the propagation of a cell or cells without terminal differentiation.

As used herein, the term "differentiation" refers to the developmental process of lineage commitment. A "lineage" refers to a pathway of cellular development, in which precursor or "progenitor" cells undergo progressive physiological changes to become a specified cell type having a characteristic function (*e.g*., nerve cell, muscle cell, or endothelial cell). Differentiation occurs in stages, whereby cells gradually become more specified until they reach full maturity, which is also referred to as "terminal differentiation." A "terminally differentiated cell" is a cell that has committed to a specific lineage, and has reached the end stage of differentiation (*i.e.,* a cell that has fully matured). By "committed" or "differentiated" is meant a cell that expresses one or more markers or other characteristic of a cell of a particular lineage.

As used herein, the term "isolated" refers to a stem cell or population of daughter stem cells in a non-naturally occurring state outside of the body (*e.g*., isolated from the body or a biological sample from the body). The biological sample can include synovial fluid, blood (*e.g*., peripheral blood), or tissue.

As used herein, the term "purified" as in a "purified cell" refers to a cell that has been separated from the body of a subject but remains in the presence of other cell types also obtained from the body of the subject. By "substantially purified" is meant that the desired cells are enriched by at least 20%, more preferably by at least 50%, even more preferably by at least 75%, and most preferably by at least 90% or even 95%.

By a "population of cells" is meant a collection of at least ten cells. Preferably, the population consists of at least twenty cells, more preferably at least one hundred cells, and most preferably at least one thousand, or even one million cells. Because the stem cells disclosed herein exhibit a capacity for self-renewal, they can be expanded in culture to produce populations of even billions of cells.

"Germ layers" are the three primary layers formed as a result of gastrulation in early stage embryos, consisting of endoderm, mesoderm, and ectoderm. Embryonic germ layers are the source from which all tissues and organs derive. The endoderm is the source of, for example, pharynx, esophagus, stomach, intestine and associated glands (*e.g*., salivary glands), liver, epithelial linings of respiratory passages and gastrointestinal tract, pancreas and lungs. The mesoderm is the source of, for example, smooth and striated muscle, connective tissue, vessels, the cardiovascular system, blood cells, bone marrow, skeleton, reproductive organs and excretory organs. Ectoderm is the source of, for example, epidermis (epidermal layer of the skin), sensory organs, the entire nervous system, including brain, spinal cord, and all the outlying components of the nervous system.

The term "multipotent," with respect to stem cells of the disclosure, refers to the ability of the stem cells to give rise to cells of all three primitive germ layers (endoderm, mesoderm, and ectoderm) upon differentiation.

The term "allogeneic," as used herein, refers to cells of the same species that differ genetically from cells of a host.

The term "autologous," as used herein, refers to cells derived from the same subject. The term "engraft" as used herein refers to the process of stem cell incorporation into a tissue of interest *in vivo* through contact with existing cells of the tissue.

By "does not detectably express" means that expression of a protein or gene cannot be detected by standard methods. In the case of cell surface markers, expression can be measured by, *e.g.,* flow cytometry, using a cut-off values as obtained from negative controls (*i.e.,* cells known to lack the antigen of interest) or by isotype controls (*i.e.,* measuring nonspecific binding of the antibody to the cell). Thus, a cell that "does not detectably express" a marker appears similar to the negative control for that marker. For gene expression, a gene "does not detectably express" if the presence of its mRNA cannot be visually detected on a standard agarose gel following standard PCR protocols.

Conversely, a cell "expresses" the protein or gene if it can be detected, including by the same method.

The term "culture expanded population" means a population of cells whose numbers have been increased by cell division *in vitro.* This term may apply to stem cell populations and non-stem cell populations alike.

The term "passaging" refers to the process of transferring a portion of cells from one culture vessel into a new culture vessel.

The term "cryopreserve" refers to preserving cells for long term storage in a cryoprotectant at low temperature.

The term "master cell bank" refers to a collection of cryopreserved cells. Such a cell bank may comprise stem cells, non-stem cells, and/or a mixture of stem cells and non-stem cells.

As used herein, the term "about" or "approximately" refers to a quantity, level, value, number, frequency, percentage, dimension, size, amount, weight or length that varies by as much as 30, 25, 20, 25, 10, 9, 8, 7, 6, 5, 4, 3, 2 or 1 % to a reference quantity, level, value, number, frequency, percentage, dimension, size, amount, weight or length. In particular aspects, the terms "about" or "approximately" when preceding a numerical value indicates the value plus or minus a range of 15%, 10%, 5%, or 1%. With respect to biological systems or processes, the term can mean within an order of magnitude, preferably within 5-fold, and more preferably within 2-fold, of a value. Unless otherwise stated, the term 'about' means within an acceptable error range for the particular value.

As used herein, the term "activated fibroblasts" refers to fibroblasts treated with one or more stimuli capable of inducing one or more alterations in the cell: metabolic, immunological, growth factor-secreting, surface marker expression, and/or production of microvesicles.

As used herein, the term "activated immune cells" refers to immune cells treated with one or more stimuli capable of inducing one or more alterations in the cell: metabolic, immunological, growth factor secreting, surface marker expression, and production of microvesicles.

The term "administered" or "administering", as used herein, refers to any method of providing a composition to an individual such that the composition has its intended effect on the patient. For example, one method of administering is by an indirect mechanism using a medical device such as, but not limited to a catheter, applicator gun, syringe *etc.* A second exemplary method of administering is by a direct mechanism such as, local tissue administration, oral ingestion, transdermal patch, topical, inhalation, suppository *etc.*

As used herein, "allogeneic" refers to tissues or cells from another body that in a natural setting are immunologically incompatible or capable of being immunologically incompatible, although from one or more individuals of the same species.

As used herein, the term "allotransplantation" refers to the transplantation of organs, tissues, and/or cells from a donor to a recipient, where the donor and recipient are different individuals, but of the same species. Tissue transplanted by such procedures is referred to as an allograft or allotransplant.

As used herein, the terms "allostimulatory" and "alloreactive" refer to stimulation and reaction of the immune system in response to an allologous antigens, or "alloantigens" or cells expressing a dissimilar HLA haplotype.

As used herein, the term "angiogenesis" refers to a physiological process involving the growth of new blood vessels from pre-existing vessels and includes initiating angiogenesis, the formation of new blood vessel by initiating from existing ones, and splitting angiogenesis (intussusception: the formation of new blood vessel by splitting off existing ones).

As used herein, the term "autoimmunity" refers to the system of immune responses of an organism against its own healthy cells and tissues.

As used herein, "autologous" refers to tissues or cells that are derived or transferred from the same individual's body (*i.e.,* autologous blood donation; an autologous bone marrow transplant).

As used herein, the term "autotransplantation" refers to the transplantation of organs, tissues, and/or cells from one part of the body in an individual to another part in the same individual, *i.e.,* the donor and recipient are the same individual. Tissue transplanted by such "autologous" procedures is referred to as an autograft or autotransplant.

The term "biologically active" or "bioactive compound" refers to any molecule having structural, regulatory or biochemical functions. For example, biological activity may be determined, for example, by restoration of wild-type growth in cells lacking protein activity. Cells lacking protein activity may be produced by many methods (*i.e.,* for example, point mutation and frame-shift mutation). Complementation is achieved by transfecting cells that lack protein activity with an expression vector that expresses the protein, a derivative thereof, or a portion thereof. In other cases, a fragment of a gene product (such as a protein) may be considered biologically active (or it may be referred to as functionally active) if it retains the activity of the full-length gene product, although it may be at a reduced but detectable level of the activity of the full-length gene product.

"Cell culture" is an artificial *in vitro* system containing viable cells, whether quiescent, senescent or (actively) dividing. In a cell culture, cells are grown and maintained at an appropriate temperature, typically a temperature of 37°C and under an atmosphere typically containing oxygen and CO2. Culture conditions may vary widely for each cell type though, and variation of conditions for a particular cell type can result in different phenotypes being expressed. The most commonly varied factor in culture systems is the growth medium. Growth media can vary in concentration of nutrients, growth factors, and the presence of other components. The growth factors used to supplement media are often derived from animal blood, such as calf serum.

As used herein, the term "collateralization" refers to the growth of a blood vessel or several blood vessels that serve the same end organ or vascular bed as another blood vessel that cannot adequately supply that end organ or vascular bed sufficiently

Throughout this specification, unless the context requires otherwise, the words "comprise", "comprises" and "comprising" will be understood to imply the inclusion of a stated step or element or group of steps or elements but not the exclusion of any other step or element or group of steps or elements. By "consisting of" is meant including, and limited to, whatever follows the phrase "consisting of." Thus, the phrase "consisting of" indicates that the listed elements are required or mandatory, and that no other elements may be present. By "consisting essentially of" is meant including any elements listed after the phrase, and limited to other elements that do not interfere with or contribute to the activity or action specified in the disclosure for the listed elements. Thus, the phrase "consisting essentially of" indicates that the listed elements are required or mandatory, but that no other elements are optional and may or may not be present depending upon whether or not they affect the activity or action of the listed elements.

As used herein, the term "conditioned medium of fibroblast regenerative cells" refers to a liquid media which has been in contact with cells, wherein said cells produce factors which enter the media, thus bestowing upon the media therapeutic activity.

The term "drug", "agent" or "compound" as used herein, refers to any pharmacologically active substance capable of being administered that achieves a desired effect. Drugs or compounds can be synthetic or naturally occurring, non-peptide, proteins or peptides, oligonucleotides, or nucleotides (DNA and/or RNA), polysaccharides or sugars.

"Growth factor" can be a naturally occurring, endogenous or exogenous protein, or recombinant protein, capable of stimulating cellular proliferation and/or cellular differentiation and/or cellular migration.

The term "subject" or "individual", as used herein, refers to a human or animal that may or may not be housed in a medical facility and may be treated as an outpatient of a medical facility. The individual may be receiving one or more medical compositions *via* the internet. An individual may comprise any age of a human or non-human animal and therefore includes both adult and juveniles (*i.e.,* children) and infants. It is not intended that the term "individual" connote a need for medical treatment, therefore, an individual may voluntarily or involuntarily be part of experimentation whether clinical or in support of basic science studies. The term "subject" or "individual" refers to any organism or animal subject that is an object of a method or material, including mammals, *e.g.,* humans, laboratory animals (*e.g.,* primates, rats, mice, rabbits), livestock (*e.g*., cows, sheep, goats, pigs, turkeys, and chickens), household pets (*e.g.,* dogs, cats, and rodents), horses, and transgenic non-human animals.

As used herein, the term "ischemia" or "ischemic condition" refers to an inadequate blood supply to an organ or part of the body. Such conditions may comprise cardiac ischemia, ischemic colitis, mesenteric ischemia, ischemic stroke, brain ischemia, renal ischemia, and limb ischemia.

"Mesenchymal stem cell" or "MSC" refers to cells that are (1) adherent to plastic, (2) express CD73, CD90, and CD105 antigens, while being CD14, CD34, CD45, and HLA-DR negative, and (3) possess ability to differentiate to osteogenic, chondrogenic and adipogenic lineage, for example. In specific aspects, mesenchymal stem cells do not express substantial (such as more than 50% compared to baseline) levels of HLA-DR, CD117, CD45, or a combination thereof. As used herein, "mesenchymal stromal cell" (which may also be referred to as "mesenchymal stem cell") or "MSC" can be derived from any tissue including, but not limited to, bone marrow, adipose tissue, amniotic fluid, endometrium, trophoblast-derived tissues, cord blood, Wharton jelly, placenta, amniotic tissue, derived from pluripotent stem cells, and tooth. As used herein, "mesenchymal stromal cell" or "MSC" includes cells that are CD34 positive upon initial isolation from tissue but are similar to cells described about phenotypically and functionally. As used herein, "MSC" includes cells that are isolated from tissues using cell surface markers selected from the list comprised of NGF-R, PDGF-R, EGF-R, IGF-R, CD29, CD49a, CD56, CD63, CD73, CD105, CD106, CD140b, CD146, CD271, MSCA-1, SSEA4, STRO-1 and STRO-3 or any combination thereof, and satisfy the ISCT criteria either before or after expansion.

As used herein, "mesenchymal stromal cell" or "MSC" includes cells described in the literature as bone marrow stromal stem cells (BMSSC), marrow-isolated adult multipotent inducible cells (MIAMI) cells, multipotent adult progenitor cells (MAPC), mesenchymal adult stem cells (MASCS), MultiStem^{®}, Prochymal^{®}, remestemcel-L, Mesenchymal Precursor Cells (MPCs), Dental Pulp Stem Cells (DPSCs), PLX cells, PLX-PAD, AlloStem^{®}, Astrostem^{®}, Ixmyelocel-T, MSC-NTF, NurOwn^{™}, Stemedyne^{™}-MSC, Stempeucel^{®}, StempeucelCLI, StempeucelOA, HiQCell, Hearticellgram-AMI, Revascor^{®}, Cardiorel^{®}, Cartistem^{®}, Pneumostem^{®}, Promostem^{®}, Homeo-GH, AC607, PDA001, SB623, CX601, AC607, Endometrial Regenerative Cells (ERC), adipose-derived stem and regenerative cells (ADRCs).

The term "pharmaceutically" or "pharmacologically acceptable", as used herein, refer to molecular entities and compositions that do not produce adverse, allergic, or other untoward reactions when administered to an animal or a human.

The term, "pharmaceutically acceptable carrier", as used herein, includes any and all solvents, or a dispersion medium including, but not limited to, water, ethanol, polyol (for example, glycerol, propylene glycol, and liquid polyethylene glycol, and the like), suitable mixtures thereof, and vegetable oils, coatings, isotonic and absorption delaying agents, liposome, commercially available cleansers, and the like. Supplementary bioactive ingredients also can be incorporated into such carriers.

The terms "reduce," "inhibit," "diminish," "suppress," "decrease," "prevent" and grammatical equivalents (including "lower," "smaller," *etc*.) when in reference to the expression of any symptom in an untreated subject relative to a treated subject, mean that the quantity and/or magnitude of the symptoms in the treated subject is lower than in the untreated subject by any amount that is recognized as clinically relevant by any medically trained personnel. In one aspect, the quantity and/or magnitude of the symptoms in the treated subject is at least 10% lower than, at least 25% lower than, at least 50% lower than, at least 75% lower than, and/or at least 90% lower than the quantity and/or magnitude of the symptoms in the untreated subject.

"Therapeutic agent" means to have "therapeutic efficacy" in modulating angiogenesis and/or wound healing and an amount of the therapeutic is said to be a "angiogenic modulatory amount", if administration of that amount of the therapeutic is sufficient to cause a significant modulation (*i.e.,* increase or decrease) in angiogenic activity when administered to a subject (*e.g*., an animal model or human patient) needing modulation of angiogenesis.

As used herein, the term "therapeutically effective amount" is synonymous with "effective amount", "therapeutically effective dose", and/or "effective dose" and refers to the amount of compound that will elicit the biological, cosmetic or clinical response being sought by the practitioner in an individual in need thereof. As one example, an effective amount is the amount sufficient to reduce immunogenicity of a group of cells. As a non-limiting example, an effective amount is an amount sufficient to promote formation of a blood supply sufficient to support the transplanted tissue. As another non-limiting example, an effective amount is an amount sufficient to promote formation of new blood vessels and associated vasculature (angiogenesis) and/or an amount sufficient to promote repair or remodeling of existing blood vessels and associated vasculature. The appropriate effective amount to be administered for a particular application of the disclosed methods can be determined by those skilled in the art, using the guidance provided herein. For example, an effective amount can be extrapolated from *in vitro* and *in vivo* assays as described in the present specification. One skilled in the art will recognize that the condition of the individual can be monitored throughout the course of therapy and that the effective amount of a compound or composition disclosed herein that is administered can be adjusted accordingly.

As used herein, the term "transplantation" refers to the process of taking living tissue or cells and implanting it in another part of the body or into another body.

"Treatment," "treat," or "treating" means a method of reducing the effects of a disease or condition. Treatment can also refer to a method of reducing the disease or condition itself rather than just the symptoms. The treatment can be any reduction from pre-treatment levels and can be but is not limited to the complete ablation of the disease, condition, or the symptoms of the disease or condition. Therefore, in the disclosed methods, "treatment" can refer to a 10%, 20%, 30%, 40%, 50%, 60%, 70%, 80%, 90%, or 100% reduction in the severity of an established disease or the disease progression, including reduction in the severity of at least one symptom of the disease. For example, a disclosed method for reducing the immunogenicity of cells is considered to be a treatment if there is a detectable reduction in the immunogenicity of cells when compared to pre-treatment levels in the same subject or control subjects. Thus, the reduction can be a 10, 20, 30, 40, 50, 60, 70, 80, 90, 100%, or any amount of reduction in between as compared to native or control levels. It is understood and herein contemplated that "treatment" does not necessarily refer to a cure of the disease or condition, but an improvement in the outlook of a disease or condition. In specific aspects, treatment refers to the lessening in severity or extent of at least one symptom and may alternatively or in addition refer to a delay in the onset of at least one symptom.

The term "selecting", or "selecting for expression" means isolated specific cell(s) from a heterogenous population of other cells, such as by utilizing one or more molecules specific to the cells.

### II. Cell Expansion

Any cells encompassed herein may be expanded in any suitable manner, including using various *in vitro* systems. In one aspect, the means of *in vitro* expansion includes one or more bioreactors. Bioreactors for use in the disclosure are designed to provide a culture process that can deliver medium and oxygenation at controlled concentrations and rates that mimic nutrient concentrations and rates *in vivo.* Bioreactors have been available commercially for many years and employ a variety of types of culture technologies. Of the different bioreactors used for mammalian cell culture, most have been designed to allow for the production of high density cultures of a single cell type. For use of the disclosure, culture techniques and bioreactors used for expansion of mesenchymal stem cells may be applied for use in regenerative fibroblasts, some means are described in references 1-8.

In one aspect, typical application of these high density systems is to produce as the end-product, a conditioned medium produced by the cells. This is the case, for example, with hybridoma production of monoclonal antibodies and with packaging cell lines for viral vector production. However, these applications differ from applications where the therapeutic end-product is the harvested cells themselves, as in some aspects of the present disclosure. Once operational, bioreactors provide automatically regulated medium flow, oxygen delivery, and temperature and pH controls, and they generally allow for production of large numbers of cells. Bioreactors thus provide economies of labor and minimization of the potential for mid-process contamination, and the most sophisticated bioreactors allow for set-up, growth, selection and harvest procedures that involve minimal manual labor requirements and open processing steps [9]. Such bioreactors optimally are designed for use with a homogeneous cell mixture or aggregated cell populations as contemplated by the present disclosure. Suitable bioreactors include but are not limited to those described in U.S. Pat. No. 5,763,194, U.S. Pat. Nos. 5,985,653 and 6,238,908, U.S. Pat. No. 5,512,480, U.S. Pat. Nos. 5,459,069, 5,763,266, 5,888,807 and 5,688,687. With any large volume, several fundamental parameters require control. Cultures must be provided with medium that allows for cell viability maintenance, proliferation and differentiation as well as final cell culture preservation. Typically, the various media are delivered to the cells by a pumping mechanism in the bioreactor, feeding and exchanging the medium on a regular basis [10]. Quantification of metabolites may be performed in order to allow for optimum culture conditions [11]. The exchange process allows for by-products to be removed from the culture.

Growing cells or tissue also requires a source of oxygen. Different cell types can have different oxygen requirements. Accordingly, a flexible and adjustable means for providing oxygen to the cells is a desired component.

Depending on the particular culture, even distribution of the cell population and medium supply in the culture chamber can be an important process control [12, 13]. Such control is often achieved by use of a suspension culture design, which can be effective where cell-to-cell interactions are not important. Examples of suspension culture systems include various tank reactor designs and gas-permeable plastic bags. For cells that do not require assembly into a three-dimensional structure or require proximity to a stromal or feeder layer (such as most blood cell precursors or mature blood cells) such suspension designs may be used. Efficient collection of the cells at the completion of the culture process is an important feature of an effective cell culture system. One approach for production of cells as a product is to culture the cells in a defined space, without physical barriers to recovery, such that simple elution of the cell product results in a manageable, concentrated volume of cells amenable to final washing in a commercial, closed system cell washer designed for the purpose [14]. In specific aspects, the system would allow for addition of a pharmaceutically acceptable carrier, with or without preservative, or a cell storage compound, as well as provide efficient harvesting into appropriate sterile packaging. In at least some cases, the harvest and packaging process may be completed without breaking the sterile barrier of the fluid path of the culture chamber [15, 16]. In some aspects, expansion of cells is performed using microcarrier beads [17-23]. In some aspects, cells are grown in media lacking xenogeneic products, referred to as "xeno-free" media. In some aspects, xeno-free media is generated using platelet lysate or other defined conditions [24-26].

In some aspects, cells are grown under hypoxic conditions in order to allow for enhanced expansion and augmented regenerative activity [27].

### III. Mitochondria

In some aspects of the disclosure, fibroblast regenerative cells, compositions or pharmaceutical formulations are augmented by the introduction or co-administration of exogenous mitochondria. Isolated and substantially pure mitochondria can be administered to fibroblast regenerative cells through various means such as lipid fusion, polyethylene glycol mediated fusion or electroporation. In other aspects of the methods of the disclosure, mitochondria may be encapsulated, or treated with agents to facilitate incorporation of said mitochondria into the methods further include administering to the subject one or more agents selected from vitamin A, vitamin C, vitamin D, vitamin E, vitamin K, folic acid, choline, vitamin B1, vitamin B2, vitamin B5, vitamin B6, vitamin B12, biotin, nicotinamide, betacarotene, coenzyme Q, selenium, superoxide dismutase, glutathione peroxide, uridine, creatine succinate, pyruvate, dihydroxyacetone), acetyl-L-carnitine, alpha-lipoic acid, cardiolipin, omega fatty acid, lithium carbonate, lithium citrate, calcium, and mixtures thereof, such as the cocktails described herein. In any of the above aspects, the mitochondria can be syngeneic mitochondria, allogeneic mitochondria, or xenogeneic mitochondria. The mitochondria for use in the methods, kits, and compositions disclosed herein can be obtained from any source described herein. Means of achieving mitochondrial transfer are known in the art and described in references 28-38. In some aspects, fibroblasts are recipients of mitochondria derived from stem cells, or cells more undifferentiated as compared to said fibroblasts as shown in references 39-50.

In certain aspects, methods include the steps of (i) separating mitochondria from other constituents of a cell to produce isolated and substantially pure mitochondria; and (ii) administering the isolated and substantially pure mitochondria into the subject, such as with the fibroblasts. In certain aspects, the cells are progenitor cells or any other cell type described herein.

### IV. Cellular Transplantation Therapy

Any medical condition, disease, or disorder may be treated by methods of the disclosure for which fibroblast regenerative cells are therapeutic or prophylactic.

As one example, autoimmune diseases are characterized by an excessive reaction of the immune system against endogenous tissue. The immune system erroneously recognizes endogenous tissue as foreign bodies to be combated. This results in severe inflammatory reactions, which lead to damage to organs affected by them. An important part in distinguishing between endogenous and exogenous structures is played by T lymphocytes or T cells, which are "trained" in the thymus to dock only onto endogenous cell surface molecules, the so-called MHC molecules, and thus to tolerate endogenous structures.

In autoimmune diseases, a group of T cells behaves abnormally. In addition to the still functioning defense from exogenous molecules and organisms, they now also attack endogenous structure. Organs or tissues are perceived as exogenous. There can be various consequences: if vital structures are affected, an autoimmune disease will take a fatal course. The immune system directs its defense against these structures, cellular and also humoral defense reactions are set in motion, and autoantibodies are formed, as a result of which the organs affected in the course of time cease to function. Most commonly, the immune system is weakened and the body becomes susceptible to all kinds of diseases. Under some circumstances, recognition of the exogenous is also disrupted, and as a result the spreading of degenerated cancer cells (for example) can no longer be effectively prevented, and those affected are more susceptible to infectious diseases. In the course of the disease, cells of the immune system destroy the endogenous structures, while the body's repair mechanisms attempt as far as possible to regenerate the damaged organ parts. As a rule, without treatment this erroneous attack of the defensive system continues throughout life or until the complete destruction of the target structure.

Autoimmune diseases are treated according to the organ affected. In this, the basic principle of the causal therapy is to suppress the activity of the immune system by administration of immunosuppressants, *e.g*., cortisone. These substances are characterized by multiple systemic side-effects and interactions, owing to which attempts have been made to develop new drugs which specifically influence the mechanisms involved in the disease event.

**In** one aspect of the disclosure, fibroblasts regenerative cells are administered to an individual for treatment of an autoimmunity or inflammatory disorder. In some aspects of the disclosure, fibroblast cells are cultured *ex vivo* and subjected to conditions that reduce their immunogenicity, and then the fibroblasts are utilized to stimulate anti-inflammatory and/or immunomodulatory properties. Additional aspects are directed to methods of administration of the cells to an individual in need thereof for the purpose of treating an autoimmune and/or inflammatory condition.

Aspects of the disclosure provide means of utilizing fibroblasts as allogeneic therapeutic cells through modification of culture conditions in order to decrease immunogenicity of the fibroblasts. In one aspect of the disclosure, fibroblasts are extracted from sources with lower immunogenicity (*e.g.* placental fibroblasts, *etc*.). In another aspect, fibroblasts are subjected to interferon gamma (IFN-gamma), such as upon culture *ex vivo,* which without being restricted to mechanism, encompasses reducing immunogenicity. The reduction in immunogenicity may be exemplified by inhibiting the ability of the fibroblasts to evoke alloreactive T cell responses, in specific aspects. In specific aspects, these modified fibroblast cells are universal donor fibroblasts.

Aspects of the present disclosure are directed to systems and methods for the use of fibroblast cells, either autologous or allogeneic, for treatment of inflammatory and/or autoimmune conditions. Methods and compositions of the disclosure encompass certain manipulated cells for the treatment of inflammatory and/or autoimmune conditions. In particular, the cells include at least fibroblasts of any kind. Means of manipulation of fibroblasts are disclosed, as well as fibroblasts of different tissue origins, which actively inhibit inflammatory and/or autoimmune processes. In one aspect of the disclosure, fibroblasts are utilized as a cellular therapy for prevention and/or treatment of autoimmune conditions. In a specific aspect, fibroblasts are treated with one or more particular agents and/or conditions to be able to directly or indirectly treat inflammatory and/or autoimmune processes. The route of administration, dosage and frequency is determined as a function of the disease process, as well as stage of the disease, and can be optimized per routine practices in medicine.

In one aspect, allogeneic fibroblasts are administered to an individual in a non-manipulated manner (for example, without prior exposure to one or more particular agents, such as interferon gamma) but selected from sources naturally characterized by immune modulatory activity, such as placental fibroblasts or adipose tissue-associated fibroblasts, for example. In other aspects of the disclosure, any fibroblasts are cultured under conditions capable of inducing retro-differentiation so as to endow an immature phenotype for the fibroblasts, wherein the immature phenotype correlates with enhanced anti-inflammatory and/or immune modulatory potential. For example, fibroblasts may be cultured in the presence of one or more histone deacetylase inhibitors, such as valproic acid (Moon *et al.,* 2008; Huang *et al.,* 2011). In addition to HDAC inhibitors, other means of inducing dedifferentiation of the fibroblasts may also be utilized in the context of the current disclosure, such as 8-Br-cAMP (Wang *et al.,* 2011); M-CSF treatment (Li *et al.,* 2016); exposure to reveresine (Li *et al.,* 2016); and/or exposure to stem cell extracts (Xiong *et al.,* 2014). Characterization of fibroblast dedifferentiation can be performed by assessment of extracellular markers, such as, such as CXCR4, VEGFR-2, CD34, and/or CD133, as well as intracellular markers such as SOX-2, NANOG, and/or OCT-4.

In one aspect of the disclosure, fibroblasts are cultured *ex vivo* using means known in the art for preserving viability and proliferative ability of fibroblasts. The disclosure provides for the modification of known culture techniques to decrease recognition of fibroblasts by the recipient immune system. In one aspect, fibroblasts are cultured in conditions that lack xenogeneic components, such as fetal calf serum. Xenogeneic components are known to trigger immunological reactions, including elicitation of antibody and T cell reactions (Selvaggi *et al.,* 1997; Mackensen *et al.,* 2000; Kadri *et al.,* 2007; Forni *et al.,* 1976; Lauer *et al.,* 1983). In many individuals, natural antibodies of the IgM isotype exist to fetal calf serum associated components (Irie *et al.,* 1974), causing rejection, inflammation or anaphylaxis subsequent to administration of cells grown in the presence of fetal calf serum (Macy *et al.,* 1989). In specific aspects, the disclosure encompasses the substitution of fetal calf serum with human platelet rich plasma, platelet lysate, umbilical cord blood serum, autologous serum, and/or defined cytokine mixes as an additional feature to reduce the immunogenicity of fibroblasts. Means of culturing tissues in xenogeneic-free medium are known in the art for other cell types (Riordan *et al.,* 2015).

Aspects of the disclosure provide methods and compositions comprising fibroblasts. Fibroblasts may be derived from various tissues or organs, such as skin, heart, blood vessels, bone marrow, skeletal muscle, liver, pancreas, brain, and/or foreskin, which can be obtained by biopsy (where appropriate) or upon autopsy. In some aspects, the cells comprise fibroblasts, which can be from a fetal, neonatal, adult origin, or a combination thereof, for example.

Various quality control means are known in the art for practitioners of the disclosure to perform clinical administration of the cells. Example criteria for qualification of the cells includes marker identification using means such as flow cytometry, viability, and/or endotoxin content, as well as assessment for microbial and mycoplasma contamination.

In one aspect of the disclosure, universal donor fibroblasts are administered to an individual for treatment of an autoimmunity or inflammatory disorder. In some aspects of the disclosure, cells are cultured *ex vivo* and subjected to conditions that reduce immunogenicity and stimulate anti-inflammatory and/or immunomodulatory properties. Additional aspects are directed to methods of administration of the cells to an individual in need thereof for the purpose of treating an autoimmune and/or inflammatory condition.

### V. Various Aspects

In accordance with the current disclosure, methods of providing a cell therapy and methods of treating a subject that would benefit from a cell therapy are provided. In one aspect, a method includes administering fibroblast regenerative cells, a population or plurality or culture of fibroblast regenerative cells, progeny of fibroblast regenerative cells or conditioned medium of fibroblast regenerative cells to the subject in an amount sufficient to provide a benefit to the subject. In particular non-limiting aspects, a subject is in need of increased, stimulated, induced, promoted, augmented or enhanced hematopoiesis. In additional non-limiting aspects, a subject is in need of increased, stimulated, induced, promoted, augmented or enhanced liver function or activity; in need of reduced, decreased, inhibited, blocked, prevented, controlled or limited inflammation or autoimmunity; or in need of increased, stimulated, induced, promoted, augmented or enhanced angiogenesis. Thus, methods of the disclosure include administering fibroblast regenerative cells, a population or plurality or culture of fibroblast regenerative cells, progeny of fibroblast regenerative cells or conditioned medium of fibroblast regenerative cells to increase, stimulate, induce, promote, augment or enhance hematopoiesis (in a deficient subject); to increase, stimulate, induce, promote, augment or enhance liver function or activity; to reduce, decrease, inhibit, block, prevent, control or limit inflammation (*e.g*., to a subject in need of inhibition of inflammation); and to increase, stimulate, induce, promote, augment or enhance angiogenesis. For example, fibroblast regenerative cells can be administered (*e.g*., intravenously) to a subject with ischemia, so as to induce angiogenesis (*e.g*., by homing to ischemic tissue in the subject). Numerous diseases have been associated with ischemia, including stroke, ischemic heart disease, liver failure, kidney failure, and peripheral artery disease. Further, methods of the disclosure include administering fibroblast regenerative cells, a population or plurality or culture of fibroblast regenerative cells, progeny of fibroblast regenerative cells or conditioned medium of fibroblast regenerative cells to treat a subject having or at risk of having ischemia in a tissue or organ (*e.g*., cardiac or pulmonary tissue, limb, or kidney); to treat a subject having or at risk of having a stroke, pulmonary fibrosis, or diabetic limb; to treat a subject in need of inhibition of fibrosis or scar tissue formation; to treat a subject having or at risk of having fibrosis or scar tissue formation in a tissue or organ (*e.g*., cardiac or pulmonary, limb, liver, pancreas, or kidney); to treat a subject in need of inhibition, reduction, decreased, controlled or reversal of pathological apoptosis; to treat a subject in need of increasing or improving a pancreas or liver function; to increase numbers or proliferation of islet cells, increase numbers or proliferation of hepatocytes, or increase insulin production; to treat a subject having or at risk of having diabetes, liver failure, cirrhosis, liver or pancreas fibrosis, or hepatitis; to treat a subject in need of osteocytes or an osteocyte function (*e.g.,* to increase, stimulate, induce, promote, augment or enhance osteocyte numbers, osteocyte formation or osteocyte function); to treat a subject having or at risk of having osteoporosis, a bone fracture or break, or is in need of a prosthesis in a joint; and to treat a subject in need of dermal stem cells, or activation or stimulation of endogenous dermal stem cells. Moreover, methods of the disclosure include administering fibroblast regenerative cells, a population or plurality or culture of fibroblast regenerative cells, progeny of fibroblast regenerative cells or conditioned medium of fibroblast regenerative cells to treat a subject in need of increased or improved pulmonary or cardiac function, for example, a subject that has or is at risk of having a cardiac or pulmonary disease. Non-limiting examples of cardiac and pulmonary diseases include artherosclerosis, myocardial infarction (Heart Attack), cardiac infection, heart failure, ischemic heart failure, high blood pressure (Hypertension), or pulmonary hypertension, idiopathic pulmonary fibrosis, stroke, congenital heart disease (CHD), congestive heart failure, angina, myocarditis, coronary artery disease, cardiomyopathy, dilated cardiomyopathy, hypertrophic cardiomyopathy, endocarditis, diastolic dysfunction, cerebrovascular disease, valve disease, mitral valve prolapse, venous thromboembolism or arrhythmia.

Additionally, methods of the current disclosure include administering fibroblast regenerative cells, a population or plurality or culture of fibroblast regenerative cells, progeny of fibroblast regenerative cells or conditioned medium of fibroblast regenerative cells to treat a subject having or at risk of having a neurological or muscular disease or disorder. Non-limiting examples of neurological and muscular diseases and disorders include multiple sclerosis (MS), spinal cord injury, muscular dystrophy (Becker's or Duchenne's), amyotrophic lateral sclerosis (ALS; Lou Gehrig's disease or classical motor neuron disease), autism, progressive bulbar palsy (progressive bulbar atrophy), pseudobulbar palsy, primary lateral sclerosis (PLS), progressive muscular atrophy, spinal muscular atrophy (SMA, including SMA type I--Werdnig-Hoffmann disease, SMA type II, or SMA type III-Kugelberg-Welander disease), Fazio-Londe disease, Kennedy disease (progressive spinobulbar muscular atrophy), congenital SMA with arthrogryposis, and post-polio syndrome (PPS).

In some aspects, methods of the current disclosure also comprise administering fibroblast regenerative cells, a population or plurality or culture of fibroblast regenerative cells, progeny of fibroblast regenerative cells or conditioned medium of fibroblast regenerative cells to treat a subject having or at risk of having an immune or inflammatory mediated disorder or disease, such as an autoimmune disease or disorder Non-limiting examples include: Thyroiditis, insulitis, multiple sclerosis, iridocyclitis, uveitis, orchitis, Addison's disease, myasthenia gravis, rheumatoid arthritis, lupus erythematosus, immune hyperreactivity, insulin dependent diabetes mellitus, anemia (aplastic, hemolytic), hepatitis, autoimmune hepatitis, skleritis, idiopathic thrombocytopenic purpura, diseases of the gastrointestinal tract (*e.g*., Crohn's disease, ulcerative colitis and other inflammatory bowel diseases), juvenile arthritis, scleroderma and systemic sclerosis, sjogren's syndrom, undifferentiated connective tissue syndrome, antiphospholipid syndrome, vasculitis (polyarteritis nodosa, allergic granulomatosis and angiitis, Wegner's granulomatosis, Kawasaki disease, hypersensitivity vasculitis, Henoch-Schoenlein purpura, Behcet's Syndrome, Takayasu arteritis, Giant cell arteritis, Thrombangiitis obliterans), polymyalgia rheumatica, essentiell (mixed) cryoglobulinemia, Psoriasis vulgaris and psoriatic arthritis, diffus fasciitis with or without eosinophilia, polymyositis and other idiopathic inflammatory myopathies, relapsing panniculitis, relapsing polychondritis, lymphomatoid granulomatosis, erythema nodosum, ankylosing spondylitis, Reiter's syndrome, inflammatory dermatitis, unwanted immune reactions and inflammation associated with arthritis, including rheumatoid arthritis, inflammation associated with hypersensitivity and allergic reactions, systemic lupus erythematosus, collagen diseases, inflammation associated with atherosclerosis, arteriosclerosis, atherosclerotic heart disease, reperfusion injury, vascular inflammatory disorders, respiratory distress syndrome or other cardiopulmonary diseases, inflammation associated with peptic ulcer, hepatic fibrosis, liver cirrhosis or other hepatic diseases, thyroiditis or other glandular diseases, glomerulonephritis or other renal and urologic diseases, otitis or other oto-rhino-laryngological diseases, dermatitis or other dermal diseases, periodontal diseases or other dental diseases, orchitis or epididimo-orchitis, infertility, orchidal trauma or other immune related testicular diseases, placental dysfunction, placental insufficiency, habitual abortion, eclampsia, pre-eclampsia and other immune and/or inflammatory-related gynaecological diseases, posterior uveitis, intermediate uveitis, anterior uveitis, conjunctivitis, chorioretinitis, uveoretinitis, optic neuritis, intraocular inflammation, *e.g.* retinitis or cystoid macular oedema, sympathetic ophthalmia, scleritis, retinitis pigmentosa, immune and inflammatory components of degenerative fondus disease, inflammatory components of ocular trauma, ocular inflammation caused by infection, proliferative vitreo-retinopathies, acute ischaemic optic neuropathy, excessive scarring, *e.g.* following glaucoma filtration operation, immune and/or inflammation reaction against ocular implants and other immune and inflammatory-related ophthalmic diseases, inflammation associated with autoimmune diseases or conditions or disorders where, both in the central nervous system (CNS) or in any other organ, immune and/or inflammation suppression would be beneficial, Parkinson's disease, complication and/or side effects from treatment of Parkinson's disease, AIDS-related dementia complex HIV-related encephalopathy, Devic's disease, Sydenham chorea, Alzheimer's disease and other degenerative diseases, conditions or disorders of the CNS, inflammatory components of strokes, post-polio syndrome, immune and inflammatory components of psychiatric disorders, myelitis, encephalitis, subacute sclerosing pan-encephalitis, encephalomyelitis, acute neuropathy, subacute neuropathy, chronic neuropathy, Guillaim-Barre syndrome, Sydenham chora, pseudo-tumour cerebri, Down's Syndrome, Huntington's disease, amyotrophic lateral sclerosis, inflammatory components of CNS compression or CNS trauma or infections of the CNS, inflammatory components of muscular atrophies and dystrophies, and immune and inflammatory related diseases, conditions or disorders of the central and peripheral nervous systems, post-traumatic inflammation, septic shock, infectious diseases, inflammatory complications or side effects of surgery or organ, inflammatory and/or immune complications and side effects of gene therapy, *e.g.* due to infection with a viral carrier, or inflammation associated with AIDS, to suppress or inhibit a humoral and/or cellular immune response, to treat or ameliorate monocyte or leukocyte proliferative diseases, *e.g.* leukaemia, by reducing the amount of monocytes or lymphocytes, for the preventing or treating graft rejection in cases of transplantation of natural or artificial cells, tissue and organs such as liver, kidney, heart, lung, cornea, bone marrow, organs, lenses, pacemakers, natural or artificial skin tissue.

Still further, methods include administering fibroblast regenerative cells, a population or plurality or culture of fibroblast regenerative cells, progeny of fibroblast regenerative cells or conditioned medium of fibroblast regenerative cells to a subject in need of stimulating, increased, inducing, augmenting, or enhanced immunological tolerance. Such methods can stimulate, increase, induce, augment, or enhance immunological tolerance thereby treating an autoimmune disorder.

Still moreover, methods of the current disclosure include administering fibroblast regenerative cells, a population or plurality or culture of fibroblast regenerative cells, progeny of fibroblast regenerative cells or conditioned medium of fibroblast regenerative cells to a subject in need of inhibiting, reducing, decreasing, blocking, preventing, controlling or limiting immunological rejection of a transplant, transplant fibrosis or graft failure. Such methods can inhibit, reduce, decrease, block, prevent, control or limit immunological rejection of the transplant, transplant fibrosis or graft failure thereby enhancing acceptance of the transplant or graft by the subject.

Additionally, methods of the current disclosure include administering fibroblast regenerative cells, a population or plurality or culture of fibroblast regenerative cells, progeny of fibroblast regenerative cells or conditioned medium of fibroblast regenerative cells to treat a subject in need of treatment for a melanoma.

Fibroblast regenerative cells can be administered or delivered to a subject by any route suitable for the treatment method or protocol. Specific non-limiting examples of administration and delivery routes include parenteral, *e.g.,* intravenous, intramuscular, intrathecal (intra-spinal), intrarterial, intradermal, subcutaneous, intra-pleural, transdermal (topical), transmucosal, intra-cranial, intra-ocular, mucosal, implantation and transplantation.

The fibroblast regenerative cells of the current disclosure can be autologous with respect to the subject, that is, the stem cells used in the method (or to produce the conditioned medium) were obtained or derived from a cell from the subject that is treated according to the method. Fibroblast regenerative cells, a population or plurality or culture of fibroblast regenerative cells, progeny of fibroblast regenerative cells or conditioned medium of fibroblast regenerative cells can be allogeneic with respect to the subject, that is, the stem cells used in the method (or to produce the conditioned medium) were obtained or derived from a cell from a subject that is different from the subject that is treated according to the method.

In one aspect of the disclosure, disclosed is a fibroblast population and various fibroblast populations that are capable of proliferating and differentiating into at least two of ectoderm, mesoderm, or endoderm, are plastic adherent, possess an enhanced proliferative activity as compared to non-selected fibroblasts, and express one or more of the following genes: Oct-4, KLF-4, Nanog, CD117, Sox-2, CD34 ,Rex-1, GDF-3, CD105, IL-10, and Stella. In another aspect, the cells are cryopreserved and the population is included within a container (*e.g.,* vial, syringe or other container suitable for local delivery into a site within a human or animal, such as a bag or other container suitable for intravenous delivery of cells within a human or animal). In another aspect, the population comprises stem cells in an amount of at least 1x10², 1x10⁶, 1x10⁹, 1x10¹⁰, 1x10¹², 1x10₁₄. In some aspects, the amount are 1x10², 1x10³, 1x10⁴, 1x10⁵, 1x10⁶, 1x10⁷, 1x10⁸, 1x10⁹, 1x10¹⁰, 1x10¹¹, 1x10¹², 1x10¹³, 1x10¹⁴, 1x10¹⁵, 1x10¹⁶, 1x10¹⁷, 1x10¹⁸, 1x10¹⁹ 1x10²⁰, 1x10²⁵, 1x10-, 1x10³⁵, 1x10⁴⁰, 1x10⁴⁵, 1x10⁵⁰, 1x10⁵⁵, 1x10⁶⁰, 1x10⁶⁵, 1x10⁷⁰, 1x10⁷⁵, 1x1080, 1x10⁸⁵, 1x10⁹⁰, 1x1095, 1x10¹⁰⁰, or any amounts in between

In another aspect, the population is contained in a 0.9% NaCl solution. In one aspect, the population further contains a bioactive compound (*e.g*., expresses a growth factor, a cytokine, an antibody or fragment thereof, or the population contains an organic molecule having a mass of less than 5,000 daltons. In another aspect, the disclosure provides a master cell bank containing a plurality of cryopreserved individually packaged populations of isolated adult stem cells, each population including at least 1x102 or more of the cells of previous aspect. The cells derived by the disclosure are capable of differentiating into various tissues, for example, the disclosure provides a method of forming a neural cell, the method involving the steps of contacting a fibroblast selected/cultured for enhanced regenerative activity under neural cell-forming conditions. In one aspect, the conditions include culturing the cell with bFGF, FGF-8, SHH, and BDNF. In another aspect, the disclosure provides a method of forming a hepatocyte, the method involving culturing a regenerative cell derived from fibroblasts under hepatocyte-forming conditions. In one aspect, the conditions include culturing the cell with hepatocyte growth factor (HGF) and FGF-4. In another aspect of the disclosure, provided is a method of forming an endothelial cell, the method involving culturing a regenerative fibroblast cell under endothelial cell-forming conditions. In one aspect, the conditions include conditions include culturing the cell with VEGF.

In another aspect, provided herein is a method of forming a hematopoietic cell, the method involving culturing a fibroblast regenerative cell under hematopoietic cell forming conditions. In one aspect, the conditions include conditions include culturing the cell with bone morphogenic protein-4 (BMP4), VEGF, bFGF, stem cell factor (SCF), Flt3L, hyper IL6, thrombopoietin (TPO) and erythropoietin (EPO).

In another aspect of the disclosure, means of treating a cardiovascular disease in a subject are disclosed, the method involving administering to the subject a fibroblast regenerative cell, or a committed or differentiated progeny thereof, in an amount sufficient to treat the disease. In one aspect, the cardiovascular disease is myocardial infarction, congestive heart failure, ischemic cardiomyopathy, and coronary artery disease. In another aspect, the disclosure provides a method of increasing vascularization in a subject, the method involving administering to the subject a fibroblast derived regenerative cell, or a committed or differentiated progeny thereof, in an amount sufficient to increase vascularization. In one aspect, the subject is suffering from type II diabetes. In another aspect, the patient is suffering of cardiovascular disease. In another aspect, the disclosure provides a method for treating a neurological disorder in a subject, the method involving administering to the subject a fibroblast derived regenerative cell or a committed or differentiated progeny thereof, in an amount sufficient to treat the disease. In one aspect, the neurological disorder is a neurodegenerative disease (*e.g.,* Parkinson's disease, Alzheimer's disease, or Huntington's disease) or neurological injury.

In another aspect, the disclosure provides a process for expanding the population of fibroblast regenerative cells involving passaging the population at least about three, four, five, six, seven, eight, nine, ten, fifteen, twenty, thirty, or forty times. In another aspect, the disclosure provides a process for differentiating the isolated fibroblast regenerative cell under conditions sufficient to differentiate the isolated stem cell.

In various aspects of the disclosure delineated herein, the antigens used as the basis of selection are CD105, CD117 and/or CD34. In other aspects, the cell is substantially purified (*e.g.,* at least about 20%, 25%, 30%, 40%, 50%, 75%, 80% or more cells. In other aspects, the cell is isolated from a mammal (*e.g.,* human). In other aspects, the cell is isolated from an adult mammal. In still other aspects, the cell contains a heterologous nucleic acid sequence.

In another aspect, the disclosure provides an isolated (*e.g.,* purified or substantially purified) fibroblast regenerative cell capable of proliferating and differentiating into ectoderm, mesoderm, and endoderm, expresses at least one of (*e.g.,* at least 2, 4, 5, or 6 of) Oct-4, Nanog, Sox-2, KLF4, c-Myc, Rex-1, GDF-3, LIF receptor, CD105, CD117, CD344, IL-10, and Stella, and does not express at least one of (*e.g.,* at least 2, 3, 4, 5, 6, 7, 8, or all of) MHC class I, MHC class II, CD45, CD13, CD49c, CD66b, , and CD90 cell surface proteins. In another aspect, the disclosure features a population of cells including cells of either of the previous two aspects. The population may contain at least 5%, 10%, 20%, 30%, 40%, 50%, 60%, 70%, 80%, 90%, 95%, or 99% of the cells of the previous two aspects (e.g., an embryoid body). The population may further be part of a composition including a cryoprotectant (*e.g.,* any described herein). In another aspect, the population of cells may be part of a composition with stem cells (*e.g.,* any known in the art such as mesenchymal cells or embryonic stem cells) or differentiated cells (*e.g.,* any described herein, including myocytes, adipocytes, ectodermal cells, muscle cells, osteoblasts, chondrocytes, endothelial cells, fibroblasts, pancreatic cells, hepatocytes, bile duct cells, bone marrow cells, neural cells, and genitourinary cells. Such cells may be autologous or allogeneic to the stem cells described herein.

In another aspect, the disclosure features a method for isolating a population of regenerative fibroblast cells. The method includes the steps (a) providing a tissue with regenerative activity; (b) enriching for a population of cells that are about 6-12 micrometers in size; and may optionally include (c) depleting cells from the population expressing stem cell surface markers or MHC proteins (*e.g.,* any described herein), thereby isolating a population of stem cells. Step (c) may include depleting cells expressing MHC class I, CD66b, glycophorin a, or glycophorin b. The method may further include (d) cryopreserving the cells. In another aspect, the method further includes (d) transfecting the cells with a polynucleotide vector containing a stem cell-specific promoter (*e.g.,* an Oct-4, Nanog, Sox-9, GDF3, Rex-1, or Sox-2 promoter, or any promoter described herein) operably linked to a reporter or selection gene; and (e) further enriching the population for the regenerative fibroblast cells using expression of the reporter or selection gene (*e.g.,* using flow cytometry). In another aspect, the method further includes (d) contacting the cells with a detectable compound (*e.g.,* carboxyfluorescein diacetate, succinimidyl ester, or Aldefluor) which enters said cells, the compound being selectively detectable in proliferating and non-proliferating cells; and (e) enriching the population of cells for the proliferating cells. In another aspect, the method further includes culturing the cells under conditions which form tissue aggregate bodies. The method may further include separating (*e.g.,* by cell depletion) cell types such as granulocytes, T-cells, B-cells, NK-cell, red blood cells, or any combination thereof, from the fibroblast regenerative cells described herein. The method may further include culturing the population of fibroblast regenerative cells under conditions which support proliferation of the cells. In any of the aspects of the disclosure, the cells may further be cryopreserved.

The disclosure also features a cell produced by any of the above methods. The disclosure also provides a method for identifying a fibroblast regenerative cell, the method including the steps of introducing into a cell a vector comprising a cell-specific promoter coupled to at least one selectable marker gene, wherein the fibroblast regenerative cell that does not express at least one of (*e.g.,* at least 2, 3, 4, 5, 6, 7, 8, or all of) MHC class I, MHC class II, CD44, CD45, CD13, CD34, CD49c, CD66b, CD73, CD105, and CD90 cell surface proteins, is capable of differentiating into mesoderm, ectoderm, and endoderm; and expresses at least one embryonic transcription factor (*e.g.,* Oct-4, Nanog, Sox-2, Rex-1, GDF-3, Stella, FoxD3, Polycomb), expressing the selectable marker gene from the cell specific promoter in said stem cell; and detecting expression of the marker gene in the stem cell, thereby identifying the cell. The method may further comprise isolating the fibroblast regenerative cell. The fibroblast cell can be derived from the bodily fluid of a mammal, such as synovial fluid or blood, as well as tissue. Preferably, the mammal is a human. In certain aspects, the cell does not express CD13, CD44, and CD90.

In various aspects, the fibroblast cell-specific promoter is an Oct-4 promoter, Nanog promoter, Sox-2 promoter, Rex-1 promoter, GDF-3 promoter, Stella promoter, FoxD3 promoter, Polycomb Repressor Complex 2 promoter, or CTCF promoter. In one aspect, the fibroblast cell-specific promoter is flanked by loxP sites. In another aspect, the vector is a retroviral vector. In yet another aspect, the selectable marker gene encodes a fluorescent protein, such as a Green Fluorescent Protein (GFP). In some aspects, enhancement of regenerative activity is provided by transfection of fibroblasts with OCT-4 as described for mesenchymal stem cells but applied to fibroblasts (Wang, K.H., et al., Upregulation of Nanog and Sox-2 genes following ectopic expression of Oct-4 in amniotic fluid mesenchymal stem cells. Biotechnol Appl Biochem, 2015. 62(5): p. 591-7).

In other aspects, cells of pluripotent ability are fused with fibroblasts to artificially generate fibroblasts with enhanced regenerative activity. Fusion of fibroblast regenerative cells with other cells are described in this reference, which the inventors seek to provide as guidance for application to fibroblasts to endow enhanced regenerative activity (Xu, D., et al., Coculturing embryonic stem cells with damaged hepatocytes leads to restoration of damage and high frequency of fusion. Cell Mol Biol (Noisy-le-grand), 2009. 55 Suppl: p. OL1186-99).

In other aspects, transfection with cell permeable NANOG [53], is applied to fibroblasts that are initially selected for expression of CD105 and/or CD117 (such as in Peitz, M., et al., Cell-permeant recombinant Nanog protein promotes pluripotency by inhibiting endodermal specification. Stem Cell Res, 2014. 12(3): p. 680-9).

In yet another aspect, a vector is utilized that comprises two selectable marker genes. In a specific aspect, the two selectable marker genes are a fluorescent protein and a protein sensitive to drug selection. In yet another aspect, the selectable marker gene encodes a cell surface protein. In another aspect, the disclosure provides a fibroblast regenerative cell isolated by a method comprising the steps of introducing into a population of fibroblasts a vector comprising a regenerative cell-specific promoter coupled to at least one selectable marker gene, wherein said regenerative cell does not express MHC class I, MHC class II, CD44, CD45, CD13, CD34, CD49c, CD73, CD105 and CD90 cell surface proteins; expressing the selectable marker gene from the regenerative-cell specific promoter in said fibroblast population; and detecting expression of the marker gene in the regenerative fibroblast cell.

In one aspect, the disclosure provides a method for the differentiation of a fibroblast regenerative cell of the disclosure into a cell lineage of a germ layer selected from the group consisting of ectoderm, mesoderm and endoderm, and/or a specific cell type including but not limited to a neural, glial, chondroblast, osteoblast, adipocyte, hepatocyte, muscle cell (*e.g.,* smooth muscle or skeletal muscle), cardiac cell, pancreatic cell, pulmonary cell, and endothelial cell.

In some aspects of the disclosure, fibroblast regenerative cells are administered together with one or more anti-inflammatory agents. Pharmaceutical agents possessing anti-inflammatory properties include Alclofenac; Alclometasone Dipropionate; Algestone Acetonide; Alpha Amylase; Alpha-lipoic acid; Alpha tocopherol; Amcinafal; Amcinafide; Amfenac Sodium; Amiprilose Hydrochloride; Anakinra; Anirolac; Anitrazafen; Apazone; Ascorbic Acid; Balsalazide Disodium; Bendazac; Benoxaprofen; Benzydamine Hydrochloride; Bromelains; Broperamole; Budesonide; Carprofen; Chlorogenic acid; Cicloprofen; Cintazone; Cliprofen; Clobetasol Propionate; Clobetasone Butyrate; Clopirac; Cloticasone Propionate; Cormethasone Acetate; Cortodoxone; Deflazacort; Desonide; Desoximetasone; Dexamethasone Dipropionate; Diclofenac Potassium; Diclofenac Sodium; Diflorasone Diacetate; Diflumidone Sodium; Diflunisal; Difluprednate; Diftalone; Dimethyl Sulfoxide; Drocinonide; Ellagic acid; Endrysone; Enlimomab; Enolicam Sodium; Epirizole; Etodolac; Etofenamate; Felbinac; Fenamole; Fenbufen; Fenclofenac; Fenclorac; Fendosal; Fenpipalone; Fentiazac; Flazalone; Fluazacort; Flufenamic Acid; Flumizole; Flunisolide Acetate; Flunixin; Flunixin Meglumine; Fluocortin Butyl; Fluorometholone Acetate; Fluquazone; Flurbiprofen; Fluretofen; Fluticasone Propionate; Furaprofen; Furobufen; Glutathione; Halcinonide; Halobetasol Propionate; Halopredone Acetate; Hesperedin; Ibufenac; Ibuprofen; Ibuprofen Aluminum; Ibuprofen Piconol; Ilonidap; Indomethacin; Indomethacin Sodium; Indoprofen; Indoxole; Intrazole; Isoflupredone Acetate; Isoxepac; Isoxicam; Ketoprofen; Lofemizole Hydrochloride; Lomoxicam; Loteprednol Etabonate; Lycopene; Meclofenamate Sodium; Meclofenamic Acid; Meclorisone Dibutyrate; Mefenamic Acid; Mesalamine; Meseclazone; Methylprednisolone Suleptanate; Morniflumate; Nabumetone; Naproxen; Naproxen Sodium; Naproxol; Nimazone; Oleuropein; Olsalazine Sodium; Orgotein; Orpanoxin; Oxaprozin; Oxyphenbutazone; Paranyline Hydrochloride; Pentosan Polysulfate Sodium; Phenbutazone Sodium Glycerate; Pirfenidone; Piroxicam; Piroxicam Cinnamate; Piroxicam Olamine; Pirprofen; Pycnogenol; Polyphenols; Prednazate; Prifelone; Prodolic Acid; Proquazone; Proxazole; Proxazole Citrate; Quercetin; Reseveratrol; Rimexolone; Romazarit; Rosmarinic acid; Rutin; Salcolex; Salnacedin; Salsalate; Sanguinarium Chloride; Seclazone; Sermetacin; Sudoxicam; Sulindac; Suprofen; Talmetacin; Talniflumate; Talosalate; Tebufelone; Tenidap; Tenidap Sodium; Tenoxicam; Tesicam; Tesimide; Tetrahydrocurcumin; Tetrydamine; Tiopinac; Tixocortol Pivalate; Tolmetin; Tolmetin Sodium; Triclonide; Triflumidate; Zidometacin; Zomepirac Sodium.

In some aspects, pharmaceutical formulations comprising fibroblast regenerative cells are provided. Pharmaceutical formulations and delivery systems appropriate for the compositions and methods are known in the art (see, *e.g.,* Remington: The Science and Practice of Pharmacy (2003) 20.sup.th ed., Mack Publishing Co., Easton, Pa.; Remington's Pharmaceutical Sciences (1990) 18.sup.th ed., Mack Publishing Co., Easton, Pa.; The Merck Index (1996) 12.sup.th ed., Merck Publishing Group, Whitehouse, N.J.; Pharmaceutical Principles of Solid Dosage Forms (1993), Technonic Publishing Co., Inc., Lancaster, Pa.; Ansel and Stoklosa, Pharmaceutical Calculations (2001) 11.sup.th ed., Lippincott Williams & Wilkins, Baltimore, Md.; and Poznansky et al., Drug Delivery Systems (1980), R. L. Juliano, ed., Oxford, N.Y., pp. 253-315).

### VI. Kits

Certain aspects of the present disclosure also concern kits containing compositions of the disclosure or compositions to implement methods of the disclosure. In some aspects, kits can be used to provide fibroblast regenerative cells, population thereof, progeny thereof or conditioned media thereof. In some cases, kits include one or more reagents for producing and/or identifying fibroblast regenerative cells.

Kits may comprise components, which may be individually packaged or placed in a container, such as a tube, bottle, vial, syringe, or other suitable container means.

Individual components may also be provided in a kit in concentrated amounts; in some aspects, a component is provided individually in the same concentration as it would be in a solution with other components. Concentrations of components may be provided as 1x, 2x, 5x, 10x, or 20x or more.

In certain aspects, negative and/or positive control agents are included in the kit. The control molecules can be used to verify the enhance regenerative activity of fibroblast cells.

Kits may comprise a container with a label. Suitable containers include, for example, bottles, vials, and test tubes. The containers may be formed from a variety of materials such as glass or plastic. The container may hold a composition which includes a probe that is useful for prognostic or non-prognostic applications, such as described above. The label on the container may indicate that the composition is used for a specific prognostic or non-prognostic application, and may also indicate directions for either *in vivo* or *in vitro* use, such as those described above. The kit may comprise the container described above and one or more other containers comprising materials desirable from a commercial and user standpoint, including buffers, diluents, filters, needles, syringes, and package inserts with instructions for use.

### EXAMPLES

The following examples are included to demonstrate certain non-limiting aspects of the disclosure. It should be appreciated by those of skill in the art that the techniques disclosed in the examples that follow represent techniques discovered by the inventors to function well in the practice of the disclosed subject matter.

### EXAMPLE 1

### ENHANCED EXPRESSION OF OCT-4 IN CD105 PURIFIED FIBROBLASTS

Fibroblasts derived from foreskin were obtained from American Type Culture Collection (ATCC) and grown in Optimem media with 10% fetal calf serum. Isolation of CD105 positive and negative cells was performed using magnetic activated cell sorting (MACS).

Briefly, suspensions of the fibroblasts were obtained from cultured cells by trypsinization. Cells were washed once with 1X PBS and resuspended with (MACS) buffer (1X PBS containing 0.5% fetal bovine serum (FBS; cat. no. SH30087.01; HyClone; GE Healthcare Life Sciences, Logan, UT, USA) and 2 mM ethylenediamine tetraacetic acid, pH 7.2). A nylon mesh was used to filter cell suspensions (30-µm pore). The cells were resuspended in MACS buffer at 10⁷ cells per 80 µl, mixed with 20 µl microbeads of directly conjugated mouse anti-human CD105 antibody (1:200; cat. no. MCA1557; Bio-Rad Laboratories, Inc., Hercules, CA, USA), and incubated at 4°C for 15 min on a rotator in the dark. Following washing in 1X PBS, the DDFCs-CD105 cells were resuspended in MACS buffer and processed in an LS+ /VS+ separation column. The column was removed from the magnetic device, and the cells were flushed out with MACS buffer. The CD105- and CD105+ cells were recovered by centrifugation at 300 x g for 10 min at 4°C.

Expression of OCT-4, a marker of stem cell potency, was assessed using flow cytometry for assessment of the intracellular OCT-4 protein. Flow cytometry was performed subsequent to the cells being stained by murine anti-OCT-4 (ab91194, Abcam, Cambridge, MA; 1:200 dilution). Secondary antibodies were Allophycocyanin anti-mouse IgG (A-865, Life Technologies, Grand Island, NY; 1:1000).

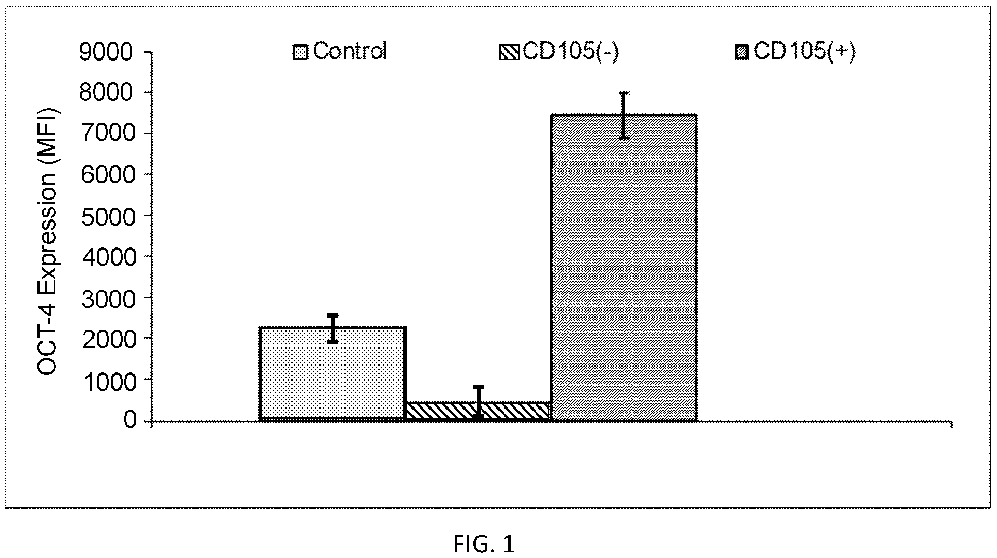

As seen in FIG.1, expression of OCT-4 was enhanced in cells that have been selected for expression of CD105.

### EXAMPLE 2

### ENHANCED EXPRESSION OF NANOG IN CD105 PURIFIED FIBROBLASTS

Fibroblasts derived from foreskin were obtained from ATCC and grown in Optimem media with 10% fetal calf serum. Isolation of CD105 positive and negative cells was performed using magnetic activated cell sorting (MACS).

Briefly, suspensions of the fibroblasts were obtained from cultured cells by trypsinization. Cells were washed once with 1X PBS and resuspended with (MACS) buffer (1X PBS containing 0.5% fetal bovine serum (FBS; cat. no. SH30087.01; HyClone; GE Healthcare Life Sciences, Logan, UT, USA) and 2 mM ethylenediamine tetraacetic acid, pH 7.2). A nylon mesh was used to filter cell suspensions (30-µm pore). The cells were resuspended in MACS buffer at 10⁷ cells per 80 µl, mixed with 20 µl microbeads of directly conjugated mouse anti-human CD105 antibody (1:200; cat. no. MCA1557; Bio-Rad Laboratories, Inc., Hercules, CA, USA), and incubated at 4°C for 15 min on a rotator in the dark. Following washing in 1X PBS, the DDFCs-CD105 cells were resuspended in MACS buffer and processed in an LS+ /VS+ separation column. The column was removed from the magnetic device, and the cells were flushed out with MACS buffer. The CD105- and CD105+ cells were recovered by centrifugation at 300 x g for 10 min at 4°C.

Expression of Nanog, a marker of potency, was assessed using flow cytometry mouse polyclonal antibody against human Nanog purchased from Abcam (Cambridge, MA).

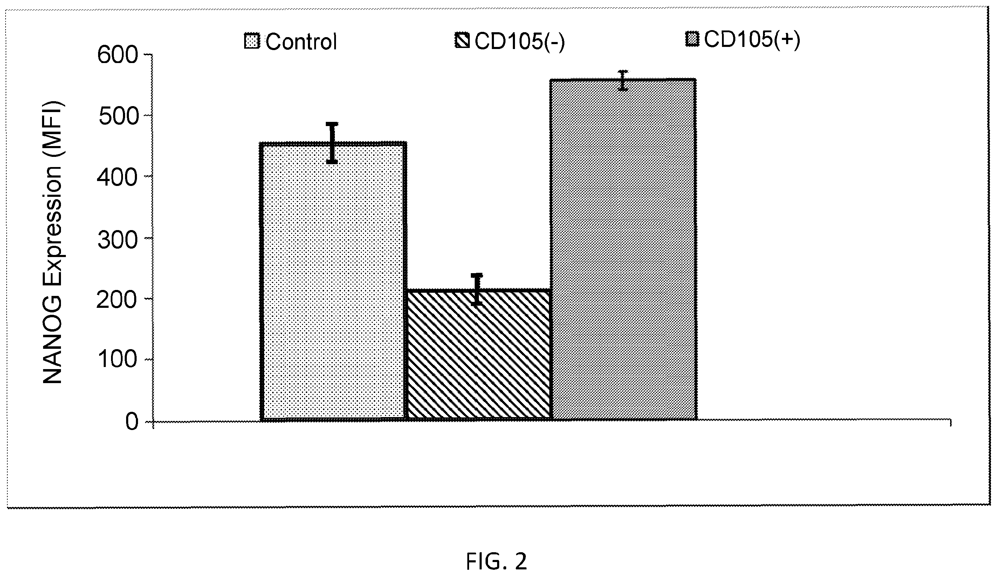

As seen in FIG. 2, expression of NANOG was enhanced in cells that have been selected for expression of CD105.

### EXAMPLE 3

### ENHANCED EXPRESSION OF KLF-4 IN CD105 PURIFIED FIBROBLASTS

Fibroblasts derived from foreskin were obtained from ATCC and grown in Optimem media with 10% fetal calf serum. Isolation of CD105 positive and negative cells was performed using magnetic activated cell sorting (MACS).

Briefly, suspensions of the fibroblasts were obtained from cultured cells by trypsinization. Cells were washed once with 1X PBS and resuspended with (MACS) buffer (1X PBS containing 0.5% fetal bovine serum (FBS; cat. no. SH30087.01; HyClone; GE Healthcare Life Sciences, Logan, UT, USA) and 2 mM ethylenediamine tetraacetic acid, pH 7.2). A nylon mesh was used to filter cell suspensions (30-µm pore). The cells were resuspended in MACS buffer at 10⁷ cells per 80 µl, mixed with 20 µl microbeads of directly conjugated mouse anti-human CD105 antibody (1:200; cat. no. MCA1557; Bio-Rad Laboratories, Inc., Hercules, CA, USA), and incubated at 4°C for 15 min on a rotator in the dark. Following washing in 1X PBS, the DDFCs-CD105 cells were resuspended in MACS buffer and processed in an LS+ /VS+ separation column. The column was removed from the magnetic device, and the cells were flushed out with MACS buffer. The CD105- and CD105+ cells were recovered by centrifugation at 300 x g for 10 min at 4°C.

Expression of Nanog, a marker of potency, was assessed using flow cytometry mouse polyclonal antibody against human KLF-4 purchased from Abcam (Cambridge, MA).

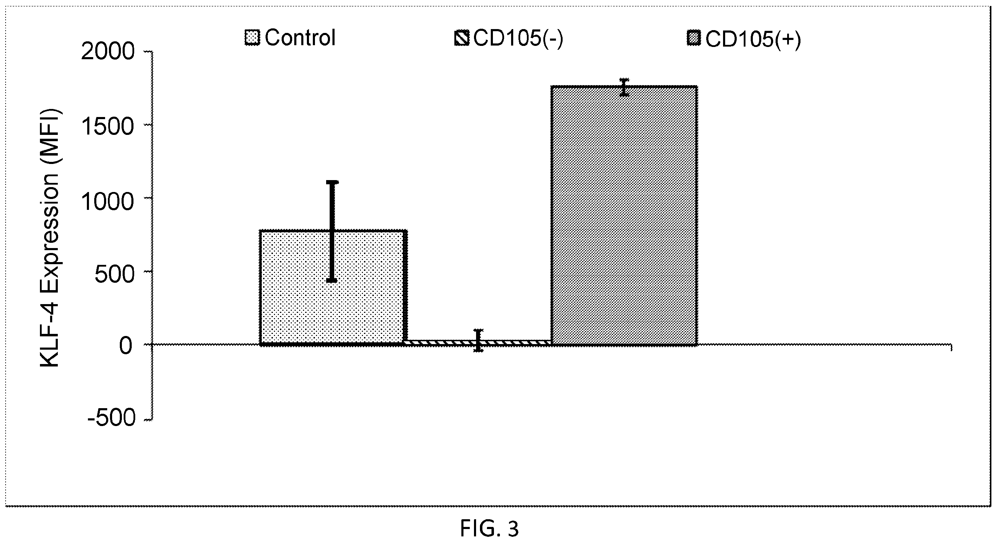

As seen in FIG. 3, expression of KLF-4 was enhanced in cells which have been selected for expression of CD105.

### EXAMPLE 4

### ENHANCED EXPRESSION OF IMMUNE MODULATORY CYTOKINE IL-10 BY CD105 SELECTED FIBROBLASTS

Fibroblasts derived from foreskin were obtained from ATCC and grown in Optimem media with 10% fetal calf serum. Isolation of CD105 positive and negative cells was performed using magnetic activated cell sorting (MACS).

Briefly, suspensions of the fibroblasts was obtained from cultured cells by trypsinization. Cells were washed once with 1X PBS and resuspended with (MACS) buffer (1X PBS containing 0.5% fetal bovine serum (FBS; cat. no. SH30087.01; HyClone; GE Healthcare Life Sciences, Logan, UT, USA) and 2 mM ethylenediamine tetraacetic acid, pH 7.2). A nylon mesh was used to filter cell suspensions (30-µm pore). The cells were resuspended in MACS buffer at 10⁷ cells per 80 µl, mixed with 20 µl microbeads of directly conjugated mouse anti-human CD105 antibody (1:200; cat. no. MCA1557; Bio-Rad Laboratories, Inc., Hercules, CA, USA), and incubated at 4°C for 15 min on a rotator in the dark. Following washing in 1X PBS, the DDFCs-CD105 cells were resuspended in MACS buffer and processed in an LS+ /VS+ separation column. The column was removed from the magnetic device, and the cells were flushed out with MACS buffer. The CD105- and CD105+ cells were recovered by centrifugation at 300 x g for 10 min at 4°C.

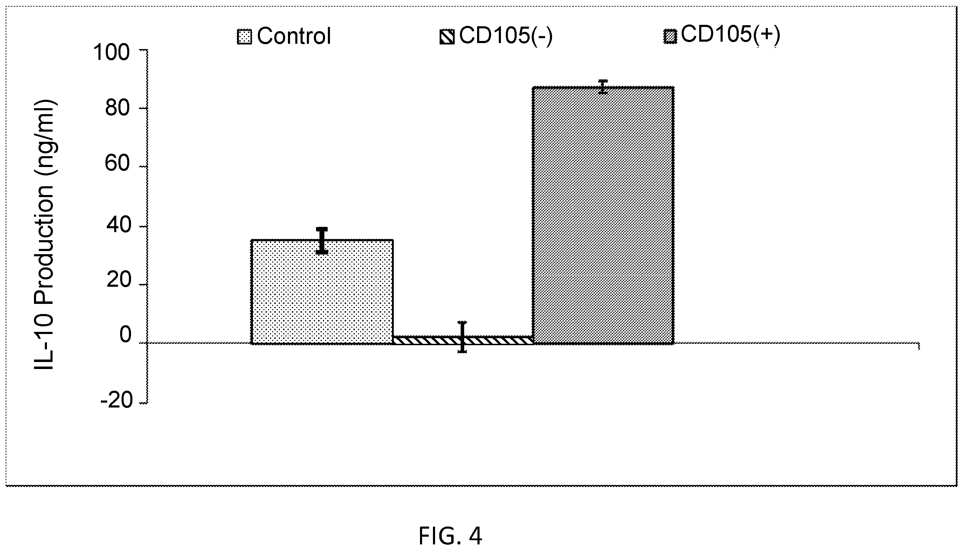

Cells were cultured with allogeneic lymphocytes at a 1:1 ratio for 72 hours and production of IL-10 was assessed by ELISA (R&D Systems). As seen in FIG. 4, an increased production of IL-10 was observed in the CD105 selected fibroblasts.

### REFERENCES

1. Jossen, V., et al., Manufacturing human mesenchymal stem cells at clinical scale: process and regulatory challenges. Appl Microbiol Biotechnol, 2018. 102(9): p. 3981-3994.
2. Sher, N. and R. Ofir, Placenta-Derived Adherent Stromal Cell Therapy for Hematopoietic Disorders: A Case Study of PLX-R18. Cell Transplant, 2018. 27(1): p. 140-150.
3. Bunpetch, V., et al., From "Bench to Bedside": Current Advancement on Large-Scale Production of Mesenchymal Stem Cells. Stem Cells Dev, 2017. 26(22): p. 1662-1673.
4. Kozanoglu, I., et al., Quantum cell expansion system: Safe and rapid expansion. Cytotherapy, 2017. 19(10): p. 1246-1247.
5. Rafiq, Q.A., et al., Process development of human multipotent stromal cell microcarrier culture using an automated high-throughput microbioreactor. Biotechnol Bioeng, 2017. 114(10): p. 2253-2266.
6. Dwarshuis, N.J., et al., Cells as advanced therapeutics: State-of-the-art, challenges, and opportunities in large scale biomanufacturing of high-quality cells for adoptive immunotherapies. Adv Drug Deliv Rev, 2017. 114: p. 222-239.
7. Panchalingam, K.M., et al., Bioprocessing strategies for the large-scale production of human mesenchymal stem cells: a review. Stem Cell Res Ther, 2015. 6: p. 225.
8. Robey, P.G., et al., Generation of clinical grade human bone marrow stromal cells for use in bone regeneration. Bone, 2015. 70: p. 87-92.
9. Petrenko, Y.A., et al., Perfusion bioreactor-based cryopreservation of 3D human mesenchymal stromal cell tissue grafts. Cryobiology, 2017. 76: p. 150-153.
10. Barckhausen, C., et al., GMP-Compliant Expansion of Clinical-Grade Human Mesenchymal Stromal/Stem Cells Using a Closed Hollow Fiber Bioreactor. Methods Mol Biol, 2016. 1416: p. 389-412.
11. Simmons, A.D., et al., Sensing metabolites for the monitoring of tissue engineered construct cellularity in perfusion bioreactors. Biosens Bioelectron, 2017. 90: p. 443-449.
12. Lambrechts, T., et al., Large-Scale Mesenchymal Stem/Stromal Cell Expansion: A Visualization Tool for Bioprocess Comparison. Tissue Eng Part B Rev, 2016. 22(6): p. 485-498.
13. Fernandes-Platzgummer, A., et al., Clinical-Grade Manufacturing of Therapeutic Human Mesenchymal Stem/Stromal Cells in Microcarrier-Based Culture Systems. Methods Mol Biol, 2016. 1416: p. 375-88.
14. Neumann, A., et al., Characterization and Application of a Disposable Rotating Bed Bioreactor for Mesenchymal Stem Cell Expansion. Bioengineering (Basel), 2014. 1(4): p. 231-245.
15. Zanetti, A., et al., Suspension-Expansion of Bone Marrow Results in Small Mesenchymal Stem Cells Exhibiting Increased Transpulmonary Passage Following Intravenous Administration. Tissue Eng Part C Methods, 2015. 21(7): p. 683-92.
16. Elseberg, C.L., D. Salzig, and P. Czermak, Bioreactor expansion of human mesenchymal stem cells according to GMP requirements. Methods Mol Biol, 2015. 1283: p. 199-218.
17. Tsai, A.C. and T. Ma, Expansion of Human Mesenchymal Stem Cells in a Microcarrier Bioreactor. Methods Mol Biol, 2016. 1502: p. 77-86.
18. Petry, F., et al., Manufacturing of Human Umbilical Cord Mesenchymal Stromal Cells on Microcarriers in a Dynamic System for Clinical Use. Stem Cells Int, 2016. 2016: p. 4834616.
19. Sart, S. and S.N. Agathos, Large-Scale Expansion and Differentiation of Mesenchymal Stem Cells in Microcarrier-Based Stirred Bioreactors. Methods Mol Biol, 2016. 1502: p. 87-102.
20. Rafiq, Q.A., et al., Systematic microcarrier screening and agitated culture conditions improves human mesenchymal stem cell yield in bioreactors. Biotechnol J, 2016. 11(4): p. 473-86.
21. Wang, X., et al., Perfusion culture-induced template-assisted assembling of cell-laden microcarriers is a promising route for fabricating macrotissues. Biotechnol J, 2014. 9(11): p. 1425-34.
22. Carmelo, J.G., et al., Scalable ex vivo expansion of human mesenchymal stem/stromal cells in microcarrier-based stirred culture systems. Methods Mol Biol, 2015. 1283: p. 147-59.
23. Hupfeld, J., et al., Modulation of mesenchymal stromal cell characteristics by microcarrier culture in bioreactors. Biotechnol Bioeng, 2014. 111(11): p. 2290-302.
24. Tozetti, P.A., et al., Expansion strategies for human mesenchymal stromal cells culture under xeno-free conditions. Biotechnol Prog, 2017. 33(5): p. 1358-1367.
25. Mizukami, A., et al., Stirred tank bioreactor culture combined with serum-/xenogeneic-free culture medium enables an efficient expansion of umbilical cord-derived mesenchymal stem/stromal cells. Biotechnol J, 2016. 11(8): p. 1048-59.
26. Carmelo, J.G., et al., A xeno-free microcarrier-based stirred culture system for the scalable expansion of human mesenchymal stem/stromal cells isolated from bone marrow and adipose tissue. Biotechnol J, 2015. 10(8): p. 1235-47.
27. Nold, P., et al., Immunosuppressive capabilities of mesenchymal stromal cells are maintained under hypoxic growth conditions and after gamma irradiation. Cytotherapy, 2015. 17(2): p. 152-62.
28. Wang, J., et al., Stem cell-derived mitochondria transplantation: a novel strategy and the challenges for the treatment of tissue injury. Stem Cell Res Ther, 2018. 9(1): p. 106.
29. Caicedo, A., et al., Artificial Mitochondria Transfer: Current Challenges, Advances, and Future Applications. Stem Cells Int, 2017. 2017: p. 7610414.
30. Melcher, M., et al., Modulation of oxidative phosphorylation and redox homeostasis in mitochondrial NDUFS4 deficiency via mesenchymal stem cells. Stem Cell Res Ther, 2017. 8(1): p. 150.
31. Morrison, T.J., et al., Mesenchymal Stromal Cells Modulate Macrophages in Clinically Relevant Lung Injury Models by Extracellular Vesicle Mitochondrial Transfer. Am J Respir Crit Care Med, 2017. 196(10): p. 1275-1286.
32. Li, C.J., et al., Enhancement of Mitochondrial Transfer by Antioxidants in Human Mesenchymal Stem Cells. Oxid Med Cell Longev, 2017. 2017: p. 8510805.
33. Jackson, M.V. and A.D. Krasnodembskaya, Analysis of Mitochondrial Transfer in Direct Co-cultures of Human Monocyte-derived Macrophages (MDM) and Mesenchymal Stem Cells (MSC). Bio Protoc, 2017. 7(9).
34. Nzigou Mombo, B., et al., MitoCeption: Transferring Isolated Human MSC Mitochondria to Glioblastoma Stem Cells. J Vis Exp, 2017(120).
35. Reznichenko, A.S., C. Huyser, and M.S. Pepper, Mitochondrial transfer: Implications for assisted reproductive technologies. Appl Transl Genom, 2016. 11: p. 40-47.
36. Kang, E., et al., Mitochondrial replacement in human oocytes carrying pathogenic mitochondrial DNA mutations. Nature, 2016. 540(7632): p. 270-275.
37. Wu, T.H., et al., Mitochondrial Transfer by Photothermal Nanoblade Restores Metabolite Profile in Mammalian Cells. Cell Metab, 2016. 23(5): p. 921-9.
38. Plotnikov, E.Y., et al., Intercellular Transfer of Mitochondria. Biochemistry (Mosc), 2015. 80(5): p. 542-8.
39. Paliwal, S., et al., Regenerative abilities of mesenchymal stem cells through mitochondrial transfer. J Biomed Sci, 2018. 25(1): p. 31.
40. Rodriguez, A.M., et al., Intercellular mitochondria trafficking highlighting the dual role of mesenchymal stem cells as both sensors and rescuers of tissue injury. Cell Cycle, 2018: p. 1-25.
41. Babenko, V.A., et al., Miro1 Enhances Mitochondria Transfer from Multipotent Mesenchymal Stem Cells (MMSC) to Neural Cells and Improves the Efficacy of Cell Recovery. Molecules, 2018. 23(3).
42. Wang, J., et al., Cell adhesion-mediated mitochondria transfer contributes to mesenchymal stem cell-induced chemoresistance on T cell acute lymphoblastic leukemia cells. J Hematol Oncol, 2018. 11(1): p. 11.
43. Guo, R., D. Davis, and Y. Fang, Intercellular transfer of mitochondria rescues virus-induced cell death but facilitates cell-to-cell spreading of porcine reproductive and respiratory syndrome virus. Virology, 2018. 517: p. 122-134.
44. Wang, Z.B., et al., Transfer of autologous mitochondria from adipose tissue-derived stem cells rescues oocyte quality and infertility in aged mice. Aging (Albany NY), 2017. 9(12): p. 2480-2488.
45. Li, X., et al., Mesenchymal stem cells alleviate oxidative stress-induced mitochondrial dysfunction in the airways. J Allergy Clin Immunol, 2017.
46. Chuang, Y.C., et al., Mitochondrial Transfer from Wharton's Jelly Mesenchymal Stem Cell to MERRF Cybrid Reduces Oxidative Stress and Improves Mitochondrial Bioenergetics. Oxid Med Cell Longev, 2017. 2017: p. 5691215.
47. Jiang, D., et al., Mitochondrial transfer of mesenchymal stem cells effectively protects corneal epithelial cells from mitochondrial damage. Cell Death Dis, 2016. 7(11): p. e2467.
48. Jackson, M.V., et al., Mitochondrial Transfer via Tunneling Nanotubes is an Important Mechanism by Which Mesenchymal Stem Cells Enhance Macrophage Phagocytosis in the In vitro and In vivo Models of ARDS. Stem Cells, 2016. 34(8): p. 2210-23.
49. Yang, H., et al., Biochip-based study of unidirectional mitochondrial transfer from stem cells to myocytes via tunneling nanotubes. Biofabrication, 2016. 8(1): p. 015012.
50. Han, H., et al., Bone marrow-derived mesenchymal stem cells rescue injured H9c2 cells via transferring intact mitochondria through tunneling nanotubes in an in vitro simulated ischemia/reperfusion model. Mol Med Rep, 2016. 13(2): p. 1517-24.
51. Wang, K.H., et al., Upregulation of Nanog and Sox-2 genes following ectopic expression of Oct-4 in amniotic fluid mesenchymal stem cells. Biotechnol Appl Biochem, 2015. 62(5): p. 591-7.
52. Xu, D., et al., Coculturing embryonic stem cells with damaged hepatocytes leads to restoration of damage and high frequency of fusion. Cell Mol Biol (Noisy-le-grand), 2009. 55 Suppl: p. OL1186-99.
53. Peitz, M., et al., Cell-permeant recombinant Nanog protein promotes pluripotency by inhibiting endodermal specification. Stem Cell Res, 2014. 12(3): p. 680-9.

## Claims

1. An *in vitro* method of producing a fibroblast regenerative cell or a population thereof, the method comprises selecting and expanding a fibroblast cell that expresses CD105 and further comprises a step of selecting the fibroblast regenerative cell for GDF-3, wherein the fibroblast cell has increased regenerative activity as compared to a control fibroblast cell that does not express CD105.

2. The *in vitro* method of claim 1, wherein the method further comprises a step of selecting the fibroblast regenerative cell for at least one additional marker selected from the group consisting of Oct-4, CD-34, KLF-4, Nanog, Sox-2, Rex-1, and Stella.

3. The *in vitro* method of claim 1 or claim 2, wherein:
a) the method further comprises a step of selecting the fibroblast regenerative cell for enhanced expression of GDF-11 as compared to a control fibroblast cell that does not express CD105; and/or
b) the fibroblast regenerative does not express at least one or more of MHC class I, MHC class II, CD45, CD13, CD49c, CD66b, CD73, CD105 or CD90 cell surface proteins; and/or
c) the fibroblast cell is derived from a tissue having regenerative properties; and/or
d) the fibroblast cell is derived from placental tissue, umbilical cord tissue, endometrial cells, Wharton's jelly, bone marrow or adipose tissue.

4. A fibroblast regenerative cell obtainable by the *in vitro* method of any one of claims 1 to 3 for use in therapy, wherein a therapeutically effective amount of the fibroblast regenerative cell, or population thereof is provided to a subject in need thereof.

5. A composition comprising an isolated fibroblast cell, or a population thereof, wherein the fibroblast cell has an increased regenerative activity compared to a control fibroblast cell, wherein the fibroblast cell expresses CD105 marker and GDF-3, and the control fibroblast cell does not express CD105.

6. The composition of claim 5, wherein:
a) the fibroblast cell further comprises a rhodamine 123 efflux activity; and/or
b) the fibroblast cell expresses at least one additional marker selected from the group consisting of Oct-4, CD-34, KLF-4, Nanog, Sox-2, Rex-1, and Stella; and/or
c) the fibroblast cell has enhanced expression of GDF-11 as compared to a control cell that does not express CD105; and/or
d) the fibroblast cell is derived from a tissue having regenerative properties; and/or
e) the fibroblast cell is derived from placental tissue, umbilical cord tissue, endometrial cells, Wharton's jelly, bone marrow or adipose tissue.

7. The composition of claim 5 or claim 6, wherein the fibroblast regenerative cell, or population thereof is cryopreserved.

8. The composition of any of claims 5 to 7, wherein:
a) the composition further comprises a bioactive compound and optionally the bioactive compound is a growth factor, a cytokine, an antibody, an antibody fragment, an organic molecule of a mass of less than 5000 daltons; and/or
b) the composition is in a NaCl solution, optionally wherein the composition is in a 0.8%-1% NaCl solution.

9. The composition of any of claims 5 to 8, wherein the composition further includes exogenous mitochondria.

10. The composition of claim 9, wherein:
a) the exogenous mitochondria have been isolated and are substantially pure from other cellular components; and/or
b) the mitochondria have been administered to the fibroblast cell by lipid fusion, polyethylene glycol-mediated fusion or by electroporation; and/or
c) the mitochondria are encapsulated or treated with agents to facilitate the incorporation of the mitochondria into the fibroblast cell; and/or
d) the composition further includes vitamin A, vitamin C, vitamin D, vitamin E, vitamin K, folic acid, choline, vitamin B1, vitamin B2, vitamin B5, vitamin B6, vitamin B12, biotin, nicotinamide, betacarotene, coenzyme Q, selenium, superoxide dismutase, glutathione peroxide, uridine, creatine succinate, pyruvate, dihydroxyacetone), acetyl-L-camitine, alpha-lipoic acid, cardiolipin, omega fatty acid, lithium carbonate, lithium citrate, calcium, or a combination thereof; and/or
e) the mitochondria are derived from stem cells, or from cells less differentiated as compared to the fibroblast cell.

11. The composition of any of claims 5 to 10, wherein the regenerative activity is the ability to stimulate angiogenesis, optionally wherein angiogenesis further comprises stimulation of human umbilical vein endothelial cell (HUVEC) proliferation and/or said angiogenesis comprises the production of collateral blood vessels.

12. The composition of claim 11, wherein:
a) said collateral blood vessels are in an ischemic cardiac tissue; or
b) said collateral blood vessels are in an ischemic limb tissue; or
c) said collateral blood vessels are surrounding an occluded blood vessel.

13. A kit comprising the composition of any of claims 5 to 12.

14. A pharmaceutical formulation comprising the composition of any of claims 5 to 12.

## Patentansprüche

1. In-vitro-Verfahren zur Herstellung einer regenerativen Fibroblastenzelle oder einer Population davon, wobei das Verfahren die Selektion und Expansion einer Fibroblastenzelle umfasst, die CD105 exprimiert, und ferner einen Schritt der Selektion der regenerativen Fibroblastenzelle für GDF-3 umfasst, wobei die Fibroblastenzelle im Vergleich zu einer Kontrollfibroblastenzelle, die CD105 nicht exprimiert, eine erhöhte regenerative Aktivität aufweist.

2. In-vitro-Verfahren nach Anspruch 1, wobei das Verfahren ferner einen Schritt der Selektion der regenerativen Fibroblastenzelle auf mindestens einen zusätzlichen Marker, ausgewählt aus der Gruppe bestehend aus Oct-4, CD-34, KLF-4, Nanog, Sox-2, Rex-1 und Stella, umfasst.

3. In-vitro-Verfahren nach Anspruch 1 oder Anspruch 2, wobei:
a) das Verfahren ferner einen Schritt der Selektion der regenerativen Fibroblastenzelle für eine verstärkte Expression von GDF-11 im Vergleich zu einer Kontrollfibroblastenzelle, die CD105 nicht exprimiert, umfasst; und/oder
b) die regenerative Fibroblastenzelle mindestens eines oder mehrere der Zelloberflächenproteine MHC-Klasse I, MHC-Klasse II, CD45, CD13, CD49c, CD66b, CD73, CD105 oder CD90 nicht exprimiert; und/oder
c) die Fibroblastenzelle aus einem Gewebe mit regenerativen Eigenschaften stammt; und/oder
d) die Fibroblastenzelle aus Plazentagewebe, Nabelschnurgewebe, Endometriumzellen, Wharton-Gelee, Knochenmark oder Fettgewebe gewonnen wird.

4. Regenerative Fibroblastenzelle, die durch das in vitro-Verfahren nach einem der Ansprüche 1 bis 3 erhältlich ist, zur Verwendung in der Therapie, wobei eine therapeutisch wirksame Menge der regenerativen Fibroblastenzelle oder einer Population davon einem Patienten, der sie benötigt, bereitgestellt wird.

5. Zusammensetzung, die eine isolierte Fibroblastenzelle oder eine Population davon umfasst, wobei die Fibroblastenzelle eine erhöhte regenerative Aktivität im Vergleich zu einer Kontrollfibroblastenzelle aufweist, wobei die Fibroblastenzelle den CD105-Marker und GDF-3 exprimiert, und die Kontrollfibroblastenzelle CD105 nicht exprimiert.

6. Zusammensetzung nach Anspruch 5, wobei:
a) die Fibroblastenzelle ferner eine Rhodamin 123-Efflux-Aktivität aufweist; und/oder
b) die Fibroblastenzelle mindestens einen zusätzlichen Marker exprimiert, ausgewählt aus der Gruppe bestehend aus Oct-4, CD-34, KLF-4, Nanog, Sox-2, Rex-1 und Stella; und/oder
c) die Fibroblastenzelle im Vergleich zu einer Kontrollzelle, die CD105 nicht exprimiert, eine erhöhte Expression von GDF-11 aufweist; und/oder
d) die Fibroblastenzelle aus einem Gewebe mit regenerativen Eigenschaften stammt; und/oder
e) die Fibroblastenzelle aus Plazentagewebe, Nabelschnurgewebe, Endometriumzellen, Wharton-Gelee, Knochenmark oder Fettgewebe gewonnen wird.

7. Zusammensetzung nach Anspruch 5 oder Anspruch 6, wobei die regenerative Fibroblastenzelle oder eine Population davon kryokonserviert ist.

8. Zusammensetzung nach einem der Ansprüche 5 bis 7, wobei:
a) die Zusammensetzung ferner eine bioaktive Verbindung umfasst und die bioaktive Verbindung optional ein Wachstumsfaktor, ein Zytokin, ein Antikörper, ein Antikörperfragment, ein organisches Molekül mit einer Masse von weniger als 5000 Dalton ist; und/oder
b) sich die Zusammensetzung in einer NaCl-Lösung befindet, optional wobei die Zusammensetzung in einer 0,8%-1%-igen NaCl-Lösung vorliegt.

9. Zusammensetzung nach einem der Ansprüche 5 bis 8, wobei die Zusammensetzung ferner exogene Mitochondrien enthält.

10. Zusammensetzung nach Anspruch 9, wobei:
a) die exogenen Mitochondrien isoliert wurden und im Wesentlichen rein von anderen zellulären Komponenten sind; und/oder
b) die Mitochondrien der Fibroblastenzelle durch Lipidfusion, Polyethylenglykol-vermittelte Fusion oder durch Elektroporation zugeführt wurden; und/oder
c) die Mitochondrien eingekapselt oder mit Mitteln behandelt sind, die den Einbau der Mitochondrien in die Fibroblastenzelle erleichtern; und/oder
d) die Zusammensetzung ferner Vitamin A, Vitamin C, Vitamin D, Vitamin E, Vitamin K, Folsäure, Cholin, Vitamin B1, Vitamin B2, Vitamin B5, Vitamin B6, Vitamin B12, Biotin, Nicotinamid, Betacarotin, Coenzym Q, Selen, Superoxiddismutase, Glutathionperoxid, Uridin, Kreatinsuccinat, Pyruvat, Dihydroxyaceton), Acetyl-L-Camitin, Alpha-Liponsäure, Cardiolipin, Omega-Fettsäure, Lithiumcarbonat, Lithiumcitrat, Calcium oder eine Kombination davon enthält; und/oder
e) die Mitochondrien von Stammzellen oder von Zellen stammen, die im Vergleich zu den Fibroblastenzellen weniger differenziert sind.

11. Zusammensetzung nach einem der Ansprüche 5 bis 10, wobei die regenerative Aktivität die Fähigkeit ist, die Angiogenese zu stimulieren, optional wobei die Angiogenese ferner die Stimulierung der Proliferation menschlicher Nabelvenenendothelzellen (Human Umbilical Vein Endothelial Cell, HUVEC) umfasst und/oder die Angiogenese die Produktion von kollateralen Blutgefäßen umfasst.

12. Zusammensetzung nach Anspruch 11, wobei:
a) die kollateralen Blutgefäße sich in einem ischämischen Herzgewebe befinden; oder
b) die kollateralen Blutgefäße sich in einem ischämischen Gliedmaßengewebe befinden; oder
c) die kollateralen Blutgefäße ein verschlossenes Blutgefäß umgeben.

13. Kit, das die Zusammensetzung nach einem der Ansprüche 5 bis 12 umfasst.

14. Pharmazeutische Formulierung, die die Zusammensetzung nach einem der Ansprüche 5 bis 12 umfasst.

## Revendications

1. Procédé *in vitro* de production d'une cellule régénératrice fibroblastique ou d'une population de celle-ci, le procédé comprend la sélection et le développement d'une cellule fibroblastique qui exprime CD105 et comprend en outre une étape de la sélection de la cellule régénératrice fibroblastique relativement à GDF-3, dans lequel la cellule fibroblastique a une activité régénérative accrue par comparaison à une cellule fibroblastique de contrôle qui n'exprime pas CD105.

2. Procédé *in vitro* de la revendication 1, dans lequel le procédé comprend en outre une étape de la sélection de la cellule régénératrice fibroblastique relativement à au moins un marqueur supplémentaire sélectionné parmi le groupe constitué de : Oct-4, CD-34, KLF-4, Nanog, Sox-2, Rex-1, et Stella.

3. Procédé *in vitro* de la revendication 1 ou la revendication 2, dans lequel :
a) le procédé comprend en outre une étape de la sélection de la cellule régénératrice fibroblastique relativement à une expression améliorée de GDF-11 par comparaison à une cellule fibroblastique de contrôle qui n'exprime pas CD105 ; et/ou
b) la cellule régénérative fibroblastique n'exprime pas au moins une ou plusieurs parmi les protéines de surface cellulaires CMH classe I, CMH classe II, CD45, CD13, CD49c, CD66b, CD73, CD105, ou CD90 ; et/ ou
c) la cellule fibroblastique est dérivée d'un tissu ayant des propriétés régénératives ; et/ou
d) la cellule fibroblastique est dérivée de : tissu placentaire, tissu de cordon ombilical, cellules endométriales, gelée de Wharton, moelle osseuse, ou tissu adipeux.

4. Cellule régénératrice fibroblastique pouvant être obtenue par le procédé *in vitro* de l'une quelconque des revendications 1 à 3 pour utilisation dans la thérapie, dans lequel une quantité thérapeutiquement efficace de la cellule régénératrice fibroblastique, ou une population de celle-ci, est fournie à un sujet en ayant besoin.

5. Composition, comprenant une cellule fibroblastique isolée, ou une population de celle-ci, dans laquelle la cellule fibroblastique a une activité régénérative accrue par comparaison à une cellule fibroblastique de contrôle, dans laquelle la cellule fibroblastique exprime le marqueur CD105, et GDF-3, et la cellule fibroblastique de contrôler n'exprime pas CD105.

6. Composition de la revendication 5, dans laquelle :
a) la cellule fibroblastique comprend en outre une activité d'efflux de rhodamine 123 ; et/ou
b) la cellule fibroblastique exprime au moins un marqueur supplémentaire sélectionné parmi le groupe constitué de : Oct-4, CD-34, KLF-4, Nanog, Sox-2, Rex-1, et Stella ; et/ou
c) la cellule fibroblastique a une expression améliorée de GDF-11 par comparaison à une cellule de contrôle qui n'exprime pas CD105 ; et/ou
d) la cellule fibroblastique est dérivée d'un tissu ayant des propriétés régénératives ; et/ou
e) la cellule fibroblastique est dérivée de : tissu placentaire, tissu de cordon ombilical, cellules endométriales, gelée de Wharton, moelle osseuse, ou tissu adipeux.

7. Composition de la revendication 5 ou la revendication 6, dans laquelle la cellule régénératrice fibroblastique, ou une population de celle-ci, est cryopréservée.

8. Composition de quelconques des revendications 5 à 7, dans laquelle :
a) la composition comprend en outre un composé bioactif et facultativement le composé bioactif est un facteur de croissance, une cytokine, un anticorps, un fragment d'anticorps, une molécule organique d'une masse de moins de 5 000 daltons ; et/ou
b) la composition est dans une solution NaCl, facultativement dans laquelle la composition est dans une solution NaCl à 0,8 % à 1 %.

9. Composition de quelconques des revendications 5 à 8, dans laquelle la composition inclut en outre des mitochondries exogènes.

10. Composition de la revendication 9, dans laquelle :
a) les mitochondries exogènes ont été isolées et sont sensiblement pures par comparaison à d'autres composants cellulaires ; et/ou
b) les mitochondries ont été administrées à la cellule fibroblastique par fusion de lipides, fusion à médiation par polyéthylène glycol, ou par électroporation ; et/ou
c) les mitochondries sont encapsulées ou traitées avec des agents pour faciliter l'incorporation des mitochondries dans la cellule fibroblastique ; et/ou
d) la composition inclut en outre : vitamine A, vitamine C, vitamine D, vitamine E, vitamine K, acide folique, choline, vitamine B1, vitamine B2, vitamine B5, vitamine B6, vitamine B12, biotine, nicotinamide, bêta-carotène, coenzyme Q, sélénium, superoxyde dismutase, peroxyde de glutathion, uridine, créatine-succinate, pyruvate, dihydroxyacétone, acétyl-L-carnitine, acide alpha-lipoïque, cardiolipine, acide gras oméga, carbonate de lithium, citrate de lithium, calcium, ou une combinaison de ceux-ci ; et/ou
e) les mitochondries sont dérivées de cellules souches, ou de cellules moins différentiées par comparaison à la cellule fibroblastique.

11. Composition de quelconques des revendications 5 à 10, dans laquelle l'activité régénérative est la capacité de stimuler l'angiogenèse, facultativement dans laquelle l'angiogenèse comprend en outre la stimulation de prolifération de cellules endothéliales de veines ombilicales humaines (HUVEC) et/ou ladite angiogenèse comprend la production de vaisseaux sanguins collatéraux.

12. Composition de la revendication 11, dans laquelle :
a) lesdits vaisseaux sanguins collatéraux sont dans un tissu cardiaque ischémique ; ou
b) lesdits vaisseaux sanguins collatéraux sont dans un tissu de membre ischémique ; ou
c) lesdits vaisseaux sanguins collatéraux entourent un vaisseau sanguin occlus.

13. Kit, comprenant la composition de quelconques des revendications 5 à 12.

14. Préparation pharmaceutique, comprenant la composition de quelconques des revendications 5 à 12.
